# EUROPEAN PATENT SPECIFICATION

(11) **EP 4 523 190 B1**
(45) Date of publication and mention of the grant of the patent: **27.08.2025**
(21) Application number: 23725243.2
(22) Date of filing: 11.05.2023
(51) Int. Cl.: G07F 11/24, G07F 11/62, G07F 17/00

(54) **METHOD, PHARMACY, DISPENSING DEVICE, MEDICATION DISPENSING CONTAINER AND COMPUTER PROGRAM PRODUCT FOR DISPENSING DISCRETE MEDICAMENTS**
VERFAHREN, PHARMAZIE, ABGABEVORRICHTUNG, ARZNEIMITTELABGABEBEHÄLTER UND COMPUTERPROGRAMMPRODUKT ZUR ABGABE EINZELNER ARZNEIMITTEL
PROCÉDÉ, PHARMACIE, DISPOSITIF DE DISTRIBUTION, RÉCIPIENT DE DISTRIBUTION DE MÉDICAMENT, ET PRODUIT PROGRAMME D'ORDINATEUR POUR DISTRIBUER DES MÉDICAMENTS DISCRETS

(30) Priority: 12.05.2022 NL 2031839
(43) Date of publication of application: 19.03.2025
(73) Proprietor: VMI Holland B.V., 8161 RK Epe (NL)
(72) Inventor: SCHEIDE, Rogier, 8161 RK Epe (NL); VAN VOORN, Patrick, 8161 RK Epe (NL)
(74) Representative: Nederlandsch Octrooibureau
(86) International application number: PCT/EP2023/062597
(87) International publication number: WO 2023/217958

(56) References cited:
- US-A1- 2009 050 694
- US-A1- 2016 068 328

## Description

### BACKGROUND

The invention relates to a method, a pharmacy, a dispensing device, a medication dispensing container and a computer program product for dispensing discrete medicaments such as pharmaceuticals, medicaments, pills, tablets or capsules for medical use.

US 10,173,830 B1 discloses a system which accommodates a series of medication dispensing containers, also known as 'feeder units' or 'canisters', for selectively dispensing an amount of said solid medications from one or more medication dispensing containers and for packing said dispensed amount. Each medication dispensing container holds an amount of solid substances specific to that respective medication dispensing container. Hence, together, the medication dispensing containers can dispense a wide variety of solid substances.

The medication dispensing container comprises an arranging body with a plurality of passageways, wherein each passageway is precisely dimensioned for accommodating said solid medications in a single row.

### SUMMARY OF THE INVENTION

For accurate counting of the dispensed medications, it is important that the solid medications are reliably singulated and dispensed one-by-one. In the system of US 10,173,830 B1, each medication has a dedicated medication dispensing container with precisely dimensioned passageways specifically for said medication. Such a dedicated medication dispensing container can effectively prevent dispensing errors such as dual dispensing.

While increasing the precision in the dimensioning of the passageways, it was found that some solid medications were repeatedly dispensed perfectly without any problems, while others occasionally experienced a failure to dispense. The cause of an occasional failure to dispense in seemingly constant and climate controlled conditions has remained a mystery. The occasional inability to dispense certain solid medications has been accepted by the industry as a problem that does not outweigh the benefits of the precise dimensioning and increased reliability of dispensing the solid medications one-by-one.

It is an object of the present invention to provide a method, a pharmacy, a dispensing device, a medication dispensing container and a computer program product for dispensing discrete medicaments, wherein the reliability of dispensing, in terms of one-by-one dispensing and/or the ability to dispense, can be improved.

According to a first aspect, the invention provides a method for dispensing discrete medicaments, wherein the method comprises the steps of:
- obtaining, by a control unit, a value of a parameter indicative of a climatological condition or variations in size and/or volume of the discrete medicaments as a result of said climatological condition, wherein the climatological condition relates to humidity and/or temperature in an environment in which the discrete medicaments are stored, filled, or dispensed;
- determining, by the control unit, if the value of the parameter is within a first range or a second range, different from the first range; and
- choosing, by the control unit, for identical discrete medicaments that are subject to hygroscopic expansion, between using a first medication dispensing container when the value of the parameter is within the first range, and using a second medication dispensing container when the value (V) of the parameter is within the second range, to dispense the identical discrete medicaments, wherein the second medication dispensing container has different dimensions than the first medication dispensing container.

The identical discrete medicaments contain hygroscopically expandable substances. In particular, the identical discrete medicaments are compressed pills or tablets. In the context of the present invention the term 'discrete medicaments' is to be interpreted as medicaments that can be dispensed one-by-one, individually, separately or in dose units.

In the context of the present invention the term 'identical discrete medicaments' is to be interpreted as: discrete medicaments being of the same brand, type and specification, composition and/or shape. The identical discrete medicaments are still considered 'identical' when subject to variations in size and/or volume as a result of hygroscopic expansion.

Moreover, it is submitted that in the context of the present invention, determining if the value of the parameter is in the first range or the second range is considered equivalent to or the same as determining if the value of the parameter is above or below a threshold value.

The Applicant has found that, despite the climate controlled environments in which the discrete medications are typically stored and/or dispensed, there are small variations in climatological conditions that can be of influence on the dimensioning of the discrete medications. In particular, it has been observed that discrete medications that contain hygroscopic substances, such as compressed pills or tablets, are susceptible to volume and/or size variations when the absolute humidity of the environment changes. Despite efforts of pharmacies to keep their environments stable in terms of temperature and relative humidity, variations in absolute humidity can still occur, in particular in different seasons throughout the year.

The method according to the present invention takes into account at least one parameter of the climatological conditions in the environment in which the discrete medicaments are stored, filled, or dispensed, and based on said at least one parameter, provides a choice or a suggestion to use the first medication dispensing container or the second medication dispensing container. The method may for example provide the choice to use of the first medication dispensing container when the value of the parameter is high and to use of the second medication dispensing container when the value of the parameter is low. The aforementioned medication dispensing containers are dimensioned to dispense the identical discrete medicaments reliably at different values of the parameter, in particular in the different ranges or above and below a chosen threshold value. In this manner, a medication dispensing container can be specifically chosen to closely match the size and/or volume of the identical discrete medicaments, with minimal tolerances, in the specific range such that the identical discrete medicaments can be dispensed more reliably, in terms of one-by-one dispensing and/or the ability to dispense, despite small variations in size and/or volume as a result of hygroscopic expansion.

By providing two different medication dispensing containers for the identical discrete medicaments, the dispensing containers themselves do not require complex mechanisms or hygroscopically expandable materials for changing the dimensions of the dispensing containers and thereby provide more reliable dispensing in a simple and cost-effective manner.

In one embodiment the method further comprises the steps of:
- filling a chosen medication dispensing container of the first medication dispensing container and the second medication dispensing container with the identical discrete medicaments; and/or
- dispensing, by a dispensing device, the identical discrete medicaments with the use of the chosen medication dispensing container.

In one embodiment the parameter is humidity, in particular the absolute humidity. The value of the parameter can thus be used as an indicator of the amount of moisture in the environment and the choice to be made to account for the hygroscopic behavior of the identical discrete medicaments at said value.

In one embodiment, the parameter is the relative humidity. The value for the absolute humidity can be obtained in at least two ways: by measuring the absolute humidity directly by a sensor or by calculating the absolute humidity based on relative humidity and a temperature associated with said relative humidity, wherein the relative humidity is directly measured by a sensor.

In one embodiment the value of the parameter is measured in a dispensing environment in which the identical discrete medicaments are dispensed. Depending on how long the identical discrete medicaments stay in the dispensing environment, it may be useful to measure the parameter in said dispensing environment such that, when the identical discrete medicaments are ultimately dispensed, the chosen medication dispensing container is optimized for reliable dispensing in the climatological conditions of the dispensing environment.

Alternatively, the value of the parameter is measured in a storage environment and/or a filling environment in which the identical discrete medicaments are stored or filled, respectively. The identical discrete medicaments may spend a considerable period of time in the storage environment and/or the filling environment. Hence, the climatological conditions of the storage environment or the filling environment may be of great influence on the hygroscopic expansion than the relatively short time spent in the dispensing environment. Thus, taking into account the climatological conditions in the storage environment and/or the filling environment may result in a better choice of which medication dispensing container to use.

In a further alternative embodiment the value of the parameter is based on or derived from meteorological data in a meteorological database. The meteorological data can be used as an indirect indicator of the climatological conditions to which the identical discrete medicaments are exposed during storage, filling or dispensing. In particular, weather models and/or weather predictions relevant for the geographical location of the pharmacy may be used to anticipate changes in the climatological conditions inside the controlled environment of the pharmacy.

In a further alternative embodiment the value of the parameter is a calendar time, in particular a calendar date, a calendar month, a calendar quarter or any other calendar related period of time. These calendar times may be used as an indirect indicator of the climatological conditions to which the identical discrete medicaments are exposed during storage, filling or dispensing. Medication dispensing containers may for example be adapted or optimized for use in specific date ranges, specific months, quarters or seasons.

In a further alternative embodiment the value of the parameter is time spent by the identical discrete medicaments in the storage environment, the filling environment, or the dispensing environment . The longer the identical discrete medicaments spent in a specific environment, the more time they will be exposed to the climatological conditions in said environment. In particular, the identical discrete medicaments may hygroscopically expand as a function of the time spent in the specific environment. Hence, a value for the time spent in said specific environment can be used to predict or estimate whether the identical discrete medicaments varied in size and/or volume by said climatological conditions and which medication dispensing container is best suited to reliably dispense the identical discrete medicaments.

In another embodiment the method comprises the step of assigning, by the control unit, the first medication dispensing container and the second medication dispensing container to the identical discrete medicaments prior to the step of choosing between the first medication dispensing container and the second medication dispensing container. Each brand, type or specification of discrete medicament typically requires a custom made medication dispensing container that has a geometry specifically adapted for reliably dispensing the specific discrete medicament. Hence, when the climatological conditions change the two medication dispensing containers can be custom made, specifically designed and/or optimized for use with the identical discrete medicaments in the specified ranges of the parameter. By assigning the medication dispensing containers to the identical discrete medicaments, the choice can be made between the medication dispensing containers available for the specific identical discrete medicaments. Optionally, the identical discrete medicaments may be part of a group of medicaments having an identical shape, yet a different composition. The first medication dispensing container and the second medication dispensing container may be assigned not only to the aforementioned identical discrete medicaments, but also to the group of medicaments having an identical shape. In this way, the assigned medication dispensing containers may not only be used for the specific identical discrete medicaments, but also to other medicaments of the group of identically shaped medicaments.

In another embodiment the choice made in the step of choosing between the first medication dispensing container and the second medication dispensing container is used to control an automated process of exchanging and filling the chosen medication dispensing container in a dispensing device or in a pharmacy housing. Alternatively, the choice can be displayed as an instruction to an operator to exchange and fill the chosen medication dispensing container with the identical discrete medicaments, for example via a human machine interface.

In another embodiment the first medication dispensing container and the second medication dispensing container each comprise a dispensing mechanism with one or more guide bodies that define dimensions of at least a part of a dispensing path of the identical discrete medicaments through said dispensing mechanism, wherein the dimensions of the dispensing path defined by said one or more guide bodies of the second medication dispensing container are different from the dimensions of the dispensing path defined by said one or more guide bodies of the first medication dispensing container. The dispensing paths of the respective medication dispensing containers can thus be custom made, specifically adapted and/or optimized for reliably dispensing the identical discrete medicaments at the respective values of the parameter for which the respective medication dispensing containers are intended.

In one embodiment thereof, the dimensions of the dispensing path defined by said one or more guide bodies are fixed for the first medication dispensing container and the second medication dispensing container. Hence, the dispensing containers themselves do not require complex mechanisms or hygroscopically expandable materials for changing the dimensions of the dispensing containers. Instead, they can be formed with a rigid or substantially rigid geometry with is less susceptible to wear or faults, and can be easier and/or less costly to produce.

According to a second aspect, the invention provides a method for dispensing discrete medicaments, wherein the method comprises the steps of:
- obtaining, by a control unit, a value of a parameter indicative of a climatological condition, wherein the climatological condition relates to humidity and/or temperature in an environment in which the discrete medicaments are stored, filled, or dispensed;
- determining if the value of the parameter is within a first range or a second range, different from the first range; and
- generating, by the control unit, an instruction for adjusting dimensions of an adjustable medication dispensing container, for identical discrete medicaments that are subject to hygroscopic expansion, wherein the adjustable medication dispensing container is adjusted to a first configuration state with first dimensions when the value of the parameter is within the first range and adjusted to a second configuration state with second dimensions, different from the first dimensions, when the value of the parameter is within the second range.

The method according to the second aspect of the invention differs from the method according to the first aspect of the invention in that a single adjustable medication dispensing container is used instead of two differently dimensioned medication dispensing containers. In this way, fewer medication dispensing containers are required to adapt to the climatological conditions, albeit at the expense of simplicity. The dimensions may be adjusted automatically or manually by an operator, for example based on instructions from the control unit. In both cases, the adjustment is made in response to the determination that is being made. Apart from that the method has the same technical advantages as the method according to the first aspect of the invention. The various embodiment of the method according to the first aspect of the invention can also be applied, *mutatis mutandis,* to the method according to the second aspect of the invention.

In one embodiment, the method further comprises the step of:
- dispensing, by a dispensing device, the identical discrete medicaments with the use of the adjustable medication dispensing container

In one embodiment the adjustable medication dispensing container comprises a dispensing mechanism with one or more guide bodies that define dimensions of at least a part of a dispensing path of the identical discrete medicaments through said dispensing mechanism, wherein the one or more guide bodies are mechanically adjustable to vary the dimensions of the dispensing path defined by said one or more guide bodies between the first configuration state and the second configuration state. The mechanical adjustment may be controlled automatically by actuators or drives, or manually by an operator.

In a further embodiment the adjustable medication dispensing container comprises an actuator element for mechanically adjusting the one or more guide bodies, wherein the actuator element comprises hygroscopically expandable material. Hence, the actuator element can respond in the same way or in a similar manner to variations in humidity as the discrete medicaments. The mechanical adjustment can thus be varied automatically.

According to a third aspect, the invention provides a pharmacy comprising a dispensing device for dispensing discrete medicaments, wherein the pharmacy comprises a set of a first medication dispensing container and a second medication dispensing container having dimensions different from the first medication dispensing container, wherein the pharmacy further comprises a control unit that is configured for:
- obtaining a value of a parameter indicative of a climatological condition, wherein the climatological condition relates to humidity and/or temperature in an environment in which the discrete medicaments are stored, filled, or dispensed;
- determining if the value of the parameter is within a first range or a second range, different from the first range; and
- choosing, for identical discrete medicaments that are subject to hygroscopic expansion, between using the first medication dispensing container when the value of the parameter is within the first range, and using the second medication dispensing container when the value of the parameter is within the second range, to dispense the identical discrete medicaments.

The control unit of the pharmacy according to the third aspect of the invention is configured for carrying out steps which are similar to the steps of the method according to the first aspect of the invention and thus has the same technical advantages, which will not be repeated hereafter.

In one embodiment the control unit, after the chosen medication dispensing container of the first medication dispensing container and the second medication dispensing container has been filled with the identical discrete medicaments, is configured for:
- controlling the dispensing device to dispense the identical discrete medicaments with the use of the chosen medication dispensing container.

In one embodiment the parameter is absolute humidity. The value for the absolute humidity can be obtained in at least two ways: the value of the parameter can be obtained by measuring the absolute humidity directly by a sensor or by calculating the absolute humidity based on relative humidity and a temperature associated with said relative humidity, wherein the relative humidity is directly measured by a sensor.

In one embodiment the pharmacy comprises a first sensor for measuring the value of the parameter in a dispensing environment in which the identical discrete medicaments are dispensed.

Alternatively, the control unit is connectable to a second sensor for measuring the value of the parameter in a storage environment or a filling environment in which the identical discrete medicaments are stored or filled, respectively.

In a further alternative embodiment the control unit is connectable to a source of meteorological data, for example, a meteorological database, wherein the value of the parameter is based on or derived from the meteorological data.

In a further alternative embodiment the control unit is configured for determining a calendar time, wherein the value of the parameter is the calendar time, in particular a calendar date, a calendar month or a calendar quarter derived from said calendar time.

In a further alternative embodiment the control unit is configured for determining a time spent by the identical discrete medicaments in a specific environment.

In another embodiment the control unit comprises a memory, wherein the control unit is configured for identifying the first medication dispensing container and the second medication dispensing container as candidates for the choice to be made, and for allocating in the memory said first medication dispensing container and said second medication dispensing container to the identical discrete medicaments.

In another embodiment the pharmacy comprises a human machine interface, wherein the control unit is operationally connected to the human machine interface for displaying the choice as an instruction to an operator on the human machine interface to fill the chosen medication dispensing container with the identical discrete medicaments.

In another embodiment the first medication dispensing container and the second medication dispensing container each comprise a dispensing mechanism with one or more guide bodies that define dimensions of at least a part of a dispensing path of the identical discrete medicaments through said dispensing mechanism, wherein the dimensions of the dispensing path defined by said one or more guide bodies of the second medication dispensing container are different from the dimensions of the dispensing path defined by said one or more guide bodies of the first medication dispensing container.

In an embodiment thereof the dimensions of the dispensing path defined by said one or more guide bodies are fixed for the first medication dispensing container and the second medication dispensing container.

According to a fourth aspect, the invention provides a pharmacy comprising a dispensing device for dispensing discrete medicaments, wherein the pharmacy comprises an adjustable medication dispensing container that is adjustable between a first configuration state with first dimensions and a second configuration state with second dimensions, different from the first dimensions, wherein the pharmacy further comprises a control unit that is configured for:
- obtaining a value of a parameter indicative of a climatological condition, wherein the climatological condition relates to humidity and/or temperature in an environment in which the discrete medicaments are stored, filled, or dispensed;
- determining if the value of the parameter is within a first range or a second range, different from the first range; and
- generating an instruction for adjusting the adjustable medication dispensing container, for identical discrete medicaments that are subject to hygroscopic expansion, wherein the adjustable medication dispensing container is adjusted to the first configuration state when the value of the parameter is within the first range and adjusted to the second configuration state when the value of the parameter is within the second range..

The control unit of the pharmacy according to the fourth aspect of the invention is configured for carrying out steps which are similar to the steps of the method according to the second aspect of the invention and thus has the same technical advantages, which will not be repeated hereafter.

In one embodiment the control unit, after the adjustment of the adjustable medication dispensing container, is configured for:
- controlling the dispensing device to dispense the identical discrete medicaments with the use of the adjustable medication dispensing container

In one embodiment the adjustable medication dispensing container comprises a dispensing mechanism with one or more guide bodies that define dimensions of at least a part of a dispensing path of the identical discrete medicaments through said dispensing mechanism, wherein the one or more guide bodies are mechanically adjustable to vary the dimensions of the dispensing path defined by said one or more guide bodies between the first configuration state and the second configuration state.

According to a fifth aspect, the invention provides a medication dispensing container for use in a pharmacy, wherein the medication dispensing container comprises a dispensing mechanism with one or more guide bodies which are mechanically adjustable between a first configuration state with first dimensions and a second configuration state with second dimensions, different from the first dimensions, wherein the medication dispensing container comprises an actuator element for mechanically adjusting the one or more guide bodies, wherein the actuator element comprises hygroscopically expandable material. This is the same medication dispensing container that is used in the method according to the second aspect of the invention and it therefore, in use, has the same technical advantages. In particular, the actuator element can respond in the same way or in a similar manner to variations in humidity as the discrete medicaments. The mechanical adjustment can thus be varied automatically.

According to a sixth aspect, the invention provides a computer program product comprising a non-transitory computer-readable medium holding instructions that, when executed by a processor, cause a pharmacy according to any one of the embodiments of the third or fourth aspect of the invention to perform the steps of the method according to any one of the embodiments of the first or second aspect of the invention. The computer program product can for example be implemented or loaded onto a control unit of the aforementioned pharmacies to carry out one of the aforementioned methods, with the previously described technical effects.

The various aspects and features described and shown in the specification can be applied, individually, wherever possible. For example, the dispensing device and the control unit of the pharmacy according to the third or fourth aspects of the invention may be applied independently of the pharmacy. These individual aspects, in particular the aspects and features described in the attached dependent claims, can be made subject of divisional patent applications.

### BRIEF DESCRIPTION OF THE DRAWINGS

The invention will be elucidated on the basis of an exemplary embodiment shown in the attached schematic drawings, in which:
figure 1 shows a pharmacy with a dispensing environment with a dispensing device, a storage environment, a filling environment, and various elements external to said pharmacy;
figure 2 shows a flow chart of a method for dispensing discrete medicaments using two medication dispensing containers according to a first embodiment of the invention;
figure 3 shows a cross section of a dispensing mechanism of the first medication dispensing container of figure 2 and an overlay of a dispensing mechanism of a second medication dispensing container having different dimensions;
figure 4 shows a cross section of an alternative adjustable medication dispensing container according to a second embodiment of the invention; and
figure 5 shows a top view of the alternative adjustable medication dispensing container according to figure 4.

### DETAILED DESCRIPTION OF THE INVENTION

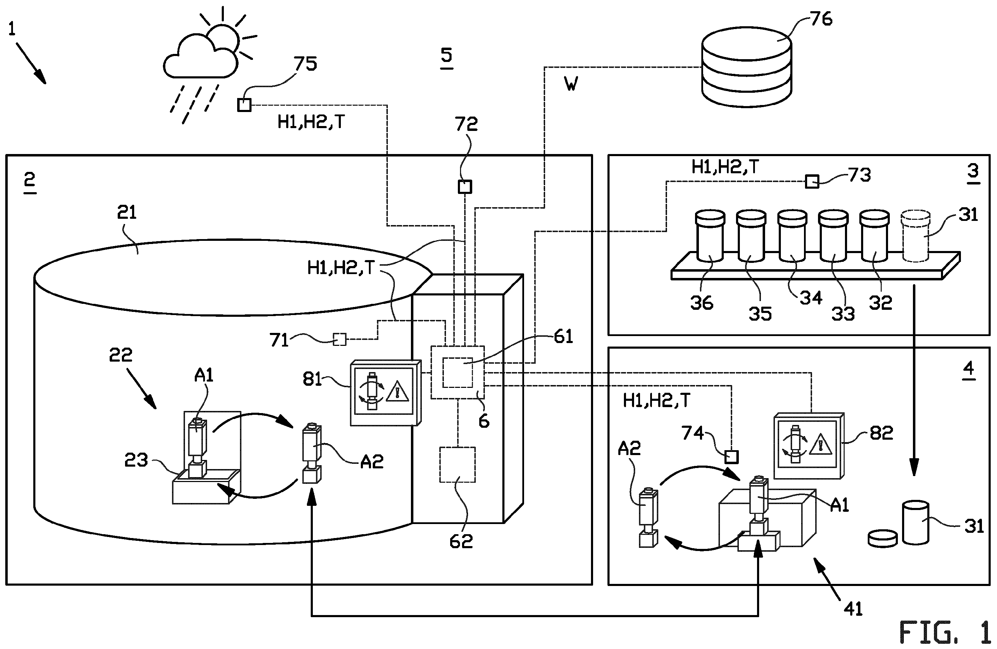

Figure 1 shows a pharmacy 1 according to a first exemplary embodiment of the invention. In the pharmacy 1, medicines, drugs, medicaments, pharmaceuticals, or other solid substances for medical use, in particular pills or tablets, are prepared for distribution to patients.

As shown in figure 1, in this example, the pharmacy 1 comprises at least one of a dispensing environment 2, a storage environment 3 and a filling environment 4. In this example, the dispensing environment 2, the storage environment 3 and/or the filing environment 4 are different environments within the same building, e.g., in one geographical location. Alternatively, the environments 2, 3, 4 may be in different or separate buildings and/or in different or separate geographical locations. The environments 2, 3, 4 may be distinct or separated from each other, for example divided by walls, or they may merely be virtually divided or not divided at all, for example when two or more of the environments 2, 3, 4 are part of the same continuous space or within the same room.

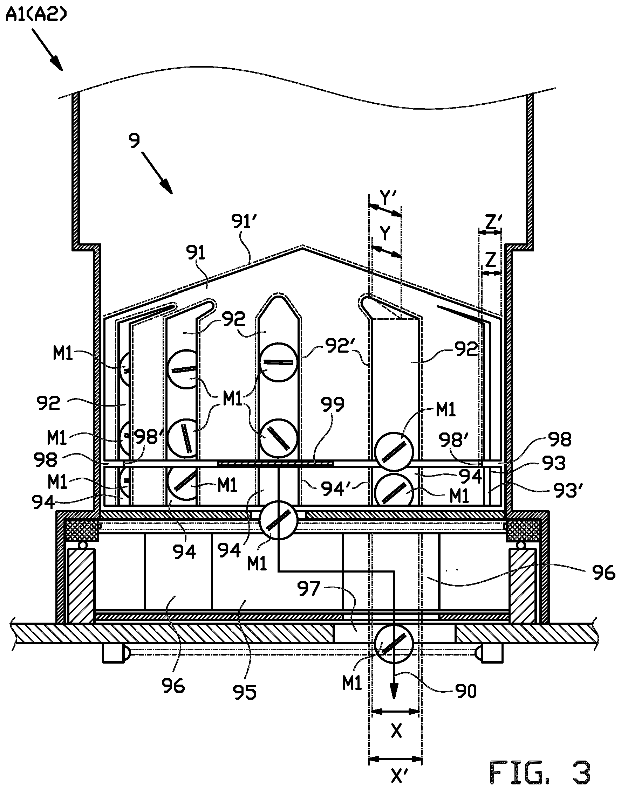

The dispensing environment 2 comprises at least one dispensing device 21 for dispensing and/or packing medicaments. The dispensing device 21 may be a largely automated system, for example corresponding to the system known from US 10,173,830 B1. The dispensing device 21 is configured for receiving medication dispensing containers A1, A2, also known as 'canisters'. As shown in figure 3, each medication dispensing container A1, A2 has a supply chamber for holding a stock of discrete medicaments, an outlet 97 and a dispensing mechanism 9, 9' for dispensing the discrete medicaments into a collection and/or packing unit of the dispensing device 21. The medicaments are discrete in the sense that they can be dispensed one-by-one, individually, separately or in dose units.

The dispensing device 21 is provided with an exchange station 22 for exchanging medication dispensing containers A1, A2, for example by replacing empty medication dispensing containers with full medication dispensing containers. In this example, the exchange station 22 is provided with an exchange drawer 23 that can be conveniently pulled out of the housing of the dispensing device 21 to facilitate the exchange.

The storage environment 3 is configured for storing stock containers 31-36 with discrete medicaments. The storage environment 3 can be configured for storing the discrete medicaments in optimal storage conditions, though the use of the current system may allow for some medicaments to be stored at conditions not previously possible and/or with medicaments that previously required other storage conditions.

The filling environment 4 is provided with a filling station 41 for filling the medication dispensing containers A1, A2 with discrete medicaments from the stock containers 31-36 of the storage environment 3.

Figure 1 further shows an external environment 5 outside of or external to the pharmacy 1. The external environment 5 is exposed to the weather and/or the climate.

The dispensing environment 2, the storage environment 3 and/or the filing environment 4 are provided with climate control, in particular air conditioning, to control climatological conditions, such as temperature and relative humidity. The climatological conditions in the dispensing environment 2, the storage environment 3 and/or the filing environment 4 may be controlled dependently or independently for each environment 2, 3, 4.

The pharmacy 1 is further provided with a control unit 6 that controls at least a part of the pharmacy's operation. In this example, the control unit 6 is part of or associated with the dispensing device 1 in the dispensing environment 2. Alternatively, the control unit 6 may be in one of the environments 3, 4 or a dedicated control environment. The control unit 6 comprises a processor 61 and a memory 62, in particular a tangible or non-transitory computer-readable medium, e.g., a physical data carrier such as a hard-drive, a USB-drive, a RAM memory or the like. The memory 62 is configured for storing computer-readable instructions that, when executed by the processor 61, causes the control unit 6 to control the operation of the pharmacy 1, and in particular the operation of the dispensing device 21. The memory 62 is further configured for storing data relevant for the aforementioned operations.

The pharmacy 1 is additionally provided with one or more sensors 71-75 for sensing or measuring a value indicative of, representing or useful for calculating or deriving parameters of climatological conditions in one or more of the environments 2, 3, 4, 5. The one or more sensors 71-75 may for example be sensors of the group comprising: humidity sensors, relative humidity sensors, absolute humidity sensors, temperature sensors or a combination thereof. Each sensor 71-75 is configured for generating and/or outputting a signal indicative of or representing a value of a climatological parameter that is measured by the respective sensor 71-75.

In particular, the one or more sensors 71-75 comprises a first sensor 71 that is located in the dispensing environment 2 within the dispensing device 1, i.e., in the same space in which the medication dispensing containers A1, A2 are positioned when they are operationally dispensing discrete medicaments. A second sensor 72 is located in the dispensing environment 2 outside of the dispensing device 21. A third sensor 73 is located in the storage environment 3. A fourth sensor 74 is located in the filling environment 4. A fifth sensor 75 is positioned in the external environment 5 outside of the pharmacy 1.

The control unit 6 is electronically and/or operationally connected to each sensor 71-75 of the one or more sensors 71-75 to obtain and process signals from said sensors 71-75. The values contained in said signals may be intermittently or continuously stored in the memory 62.

Additionally or alternatively, the control unit 6 can be electronically and/or operationally connected to a source of meteorological data, for example a meteorological database 76. The meteorological database 76 can provide information about climatological conditions related to the geographical location of the pharmacy 1 and/or the meteorological season, based on real-time or historical data. The meteorological database 76 may be located in, at or near the pharmacy 1, or it may be remotely accessible, for example via the internet.

Additionally or alternatively, the control unit 6 may be configured for obtaining a value for a calendar time, such as a calendar date, a calendar month and/or a calendar quarter, for example from an internal clock or via a network time protocol.

As shown in figure 1, the pharmacy 1 further comprises one or more human machine interfaces 81, 82, for example a display or a screen, which are electronically or operationally connected to the control unit 6 for feeding back information processed by the processor 61 to an operator, for example a pharmacist, in the pharmacy 1. In this example, a first human machine interface 81 of the one or more human machine interfaces 81, 82 is provided in the dispensing environment 2, in particular at or associated with the dispensing device 21, and a second human machine interface 82 of the one or more human machine interfaces 81, 82 is provided in the filling environment 2, in particular at or associated with the filling station 41.

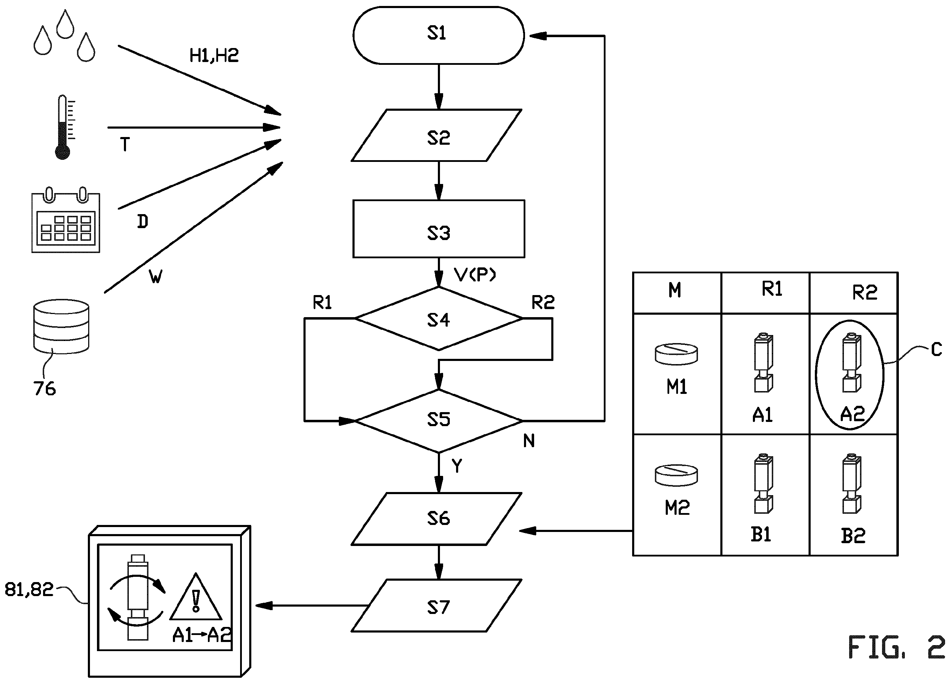

Figure 2 shows a flowchart of steps of a method for dispensing discrete medicaments M1, M2, using the pharmacy 1 according to figure 1.

Figure 2 shows two different discrete medicaments M1, M2 which are subject to hygroscopic expansion, in particular variations in size and/or volume as a result of hygroscopic expansion, meaning that they increase in size and/or volume when absorbing moisture. More in particular, the first discrete medicament M1 as shown in representative of identical discrete medicaments M1 that are 'identical' to each other in the sense that they are of the same certain brand, type, specification, composition and/or shape. The identical first discrete medicaments M1 are still considered 'identical' when variations in size and/or volume as a result of the hygroscopic expansion occur. Similarly, the second discrete medicament M2 as shown is representative of identical discrete medicaments M2 different from the first discrete medicament M1, but 'identical' to each other apart from variations in size and/or volume as a result of the hygroscopic expansion.

When the method is initiated (Step S1), information, data or signals are transmitted and/or measured by the one or more sensors 71-75 and/or the meteorological database 76 of figure 1, and obtained and processed by the control unit 6 of figure 1 (Step S2). The signals are then converted into a value V of a parameter P that is indicative of a climatological condition (Step S3). The signals may be converted directly into said value V, or the signals may be used to indirectly calculate or derive the value V of said parameter P.

The parameter P may for example be related to humidity H1, in particular absolute humidity H1. The value V of the absolute humidity H1 can be obtained by measuring the absolute humidity H1 directly. Alternatively, the value V of the absolute humidity H1 can be obtained indirectly by calculating the absolute humidity H1 based on relative humidity H2 and a temperature T associated with said relative humidity H2, for example using the ideal gas equation.

The parameter P may alternatively be a calendar time D, in which case the value V may for example be a calendar date, a calendar month, a calendar quarter or another calendar related period of time.

The parameter P may alternatively be a value for a time spent by the discrete medicaments M1, M2 in a specific environment, for example in the dispensing environment 2, the storage environment 3 and/or the filling environment 4.

The parameter P may also be meteorological data W obtained from the meteorological database, in which case the value V may be meteorological season or a meteorological parameter. The value V of the meteorological data W may be indicative of real-time climatological conditions or it may be based on historical data. The meteorological data W for example includes the absolute humidity H1, the relative humidity H2 and/or the temperature T for the relevant geographical location of the pharmacy 1 and/or for the relevant calendar time D or meteorological season.

In the next step (Step S4) it is determined if the value V of the parameter P is within a first range R1 or a second range R2, different from the first range R1. In particular it is determined if the value V of the parameter P is above or below a threshold value that separates the two ranges R1, R2. The ranges R1, R2 and/or the threshold value are stored in the memory 62 of the control unit 6 of figure 1. In practice, each range R1, R2 indicates whether a variation in size and/or volume of the discrete medicaments M1, M2 is expected as a result of hygroscopic expansion. For example, if the value V of the parameter P is within a second range R2, and the range R2 indicates an increase in the volume of the discrete medicaments, a dispensing container with larger dimensions will be chosen.

In case of the parameter P being the absolute humidity H1, the two ranges R1, R2 may be chosen to correspond to distinct ranges of said absolute humidity H1, or a specific threshold value may be chosen for said absolute humidity H1, for example sixty percent.

In case of the parameter P being a calendar time D, each range may be chosen to correspond to a chosen calendar range, for example a date range, a month range or a quarter range, or the threshold value may be a chosen date, a chosen month or a chosen quarter, for example September or the third quarter. The calendar time D is considered indicative of a climatological condition because it can be used to determine which part of the annual climatological cycle is current. Based on historical data of the annual climatological cycle, one can determine which climatological conditions typically apply without knowing or measuring the climatological conditions directly or in real-time.

In case of the parameter P being the time spent in a certain environment, the range R1, R2 may be separated by a threshold value of the time spent. For example a number of hours, a number of days, a number of weeks or a number of months in the certain environment. The threshold value may be chosen based on how the climatological conditions of the specific environment change the size and/or volume of the discrete medicaments M1, M2 over time. For example, if the absolute humidity H1 is relatively high, the threshold value will be chosen at a lower value compared to a situation in which the absolute humidity H1 is relatively low.

In case of the parameter P being derived from the meteorological data W, each range R1, R2 may be chosen to correspond to a range of a meteorological parameter, such as the absolute humidity H1, the relative humidity H2 and/or the temperature T, or to a chosen meteorological season, for example Summer or Winter, or the threshold value may be at a transition between the meteorological seasons. Again, merely knowing the current meteorological season is enough to determine which climatological conditions apply to each range R1, R2 without knowing or measuring the climatological conditions directly or in real-time.

The next step (Step S5) is optional and is used to determine if the determined range R1, R2 is different from a range R1, R2 that was determined during a previously cycle of the method (if any). If 'yes' (Y), the method continues to the next step (Step S6). If 'no' (N), the method returns to the start of the method (Step S1) and the steps (S1 to S4) are repeated.

Subsequently, the method comprises the step (Step S6) of looking up and/or choosing the medication dispensing container A1, A1, B1, B2 best suited for a specific discrete medicament M1, M2 in the previously determined range R1, R2. For this purpose, for certain discrete medicaments M1, M2 at least two different medication dispensing containers A1, A2, B1, B2 are assigned to the ranges R1, R2 for each discrete medicament M1, M2 and allocated in the memory 62.

More in particular, as shown in figure 2, the medication dispensing containers A1, A2, B1, B2 may for example be listed in a table in two columns representing the ranges R1, R2 and linked in rows to the discrete medicaments M1, M2 to which the medication dispensing containers A1, A2, B1, B2 have been assigned by the control unit 6. In this example, a first discrete medicament M1 has been assigned a first medication dispensing container A1 and a second medication dispensing container A2 for the first range R1 and the second range R2, respectively. Similarly, a second discrete medicament M2, different from the first discrete medicament M1, has been assigned an alternative first medication dispensing container B1 and an alternative second medication dispensing container B2 for the first range R1 and the second range R2, respectively.

The choice C of the best suited medication dispensing container A1, A2, B1, B2 for a specific discrete medicament M1, M2 is highlighted schematically in figure 2 with circle C. The choice C may be displayed as a preference or recommendation to the pharmacist, with the pharmacist making or confirming the final choice.

In the final step (Step S7) of the method, the choice C may be displayed for the pharmacist, with the pharmacist making or confirming a final choice. Alternatively, the choice is converted into an instruction and is displayed to the pharmacist to exchange the medication dispensing container A1 that is currently in the dispensing device 21 of figure 1 with the chosen medication dispensing container A2, and/or to fill the chosen medication dispensing container A2 with the identical first discrete medicaments M1. The choice C may be output, and/or displayed on one of the human machine interfaces 81, 82, for example at the dispensing device 21 or the filling station 41. Alternatively, when the exchange and/or filling of medication dispensing containers A1, A2, B1, B2 can be at least partially automated, e.g., the instruction may be fed directly to the corresponding automated machinery to execute the exchange or the filling operation.

In summary, the method involves choosing, for identical discrete medicaments M1 that are subject to variations in size and/or volume as a result of hygroscopic expansion, between the first medication dispensing container A1 when the value V of the parameter P is within the first range R1 and the second medication dispensing container A2 when the value V of the parameter P is within the second range R2.

As shown in figure 3, the geometry of the second medication dispensing container A2 (shown in dashed lines) is different from the geometry of the first medication dispensing container A1 (shown in solid lines). In particular, the second medication dispensing container A2 has different dimensions than the first medication dispensing container A1.

The first medication dispensing container A1 is provided with a dispensing mechanism 9, the operation of which is already extensively described in US 10,173,830 B1, which is hereby incorporated by reference, and only briefly recited hereafter.

The dispensing mechanism 9 comprises a first guide body 91 in the form of a supply body that prepares the identical first discrete medicaments M1 in one or more supply passageways 92. The dispensing mechanism 9 further comprises a second guide body 93 in the form of a singulating body with one or more singulating chambers 94 for receiving a single first discrete medicament M1 or a dose amount of the identical first discrete medicaments M1 from the one or more supply passageways 92. The dispensing mechanism 9 further comprises a third guide body 95 in the form of a dispensing body having one or more waiting chambers 96. One or more of the guide bodies 91, 93, 95 are configured to be rotatable for supplying, singulating and dispensing the first discrete medicaments M1.

In this example, the dispensing mechanism 9 further comprises a circumferential recess 98 between the first guide body 91 and the second guide body 93 for receiving a separation member 99, for example a separation plate or a brush, that separates the supply passageway 92 from the singulating chamber 94 in a position where the first discrete medicaments M1 are transferred from the singulating chamber 94 to the waiting chamber 96.

The guide bodies 91, 93, 95 and the brush recess 98 define dimensions X, Y and Z of at least a part of a dispensing path 90 of the identical first discrete medicaments M1 through said dispensing mechanism 9. In particular, the first guide body 91 and the second guide body 92 defines a width X and a depth Y of the one or more supply passageways 92 and/or the one or more singulating chambers 94. The circumferential recess 98 defines a depth Z of the circumferential recess and determines the dimensions of the separation member 99 received therein.

Other dimensions may also be defined by parts of the dispensing mechanism 9 including, but not limited to: the height of the one or more singulating chamber 94, the width, depth and/or height of the one or more waiting chambers 96, and the oblique angle of the cone shaped upper surface of the first guide body 91.

The second medication dispensing container A2, shown in dashed lines in figure 3, has an alternative dispensing mechanism 9' with one or more alternative guide bodies 91', 93' and/or an alternative dimensioned circumferential recess 98', resulting in different dimensions X', Y', Z' for the one or more supply passageways 92', the one or more singulating chambers 94', and/or the circumferential recess 98'.

In this manner, the chosen medication dispensing container A1, A2 can be specifically adapted to closely match the size and/or volume of the identical first discrete medicaments M1, i.e. with minimal tolerances, in the specific range R1, R2 such that the identical first discrete medicaments M1 can be dispensed more reliably, in terms of one-by-one dispensing and/or the ability to dispense, despite small variations as a result of hygroscopic expansion.

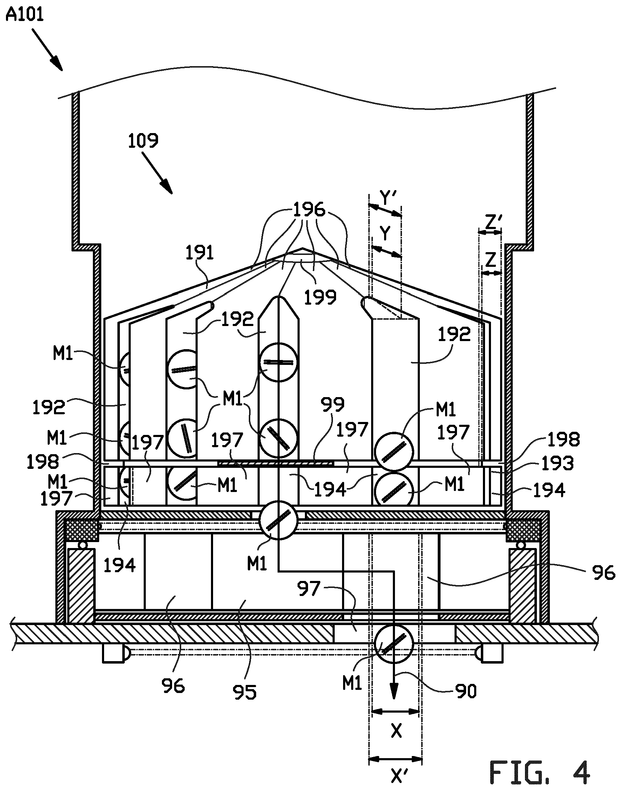

Figure 4 shows an alternative adjustable medication dispensing container A101 that differs from the aforementioned first medication dispensing container A1 in that it comprises an alternative dispensing mechanism 109 with one or more guide bodies 191, 193 and a circumferential recess 198 which are mechanically adjustable to vary the dimensions X, X'; Y, Y'; Z, Z' of the dispensing path 190 between a first configuration state and a second configuration state.

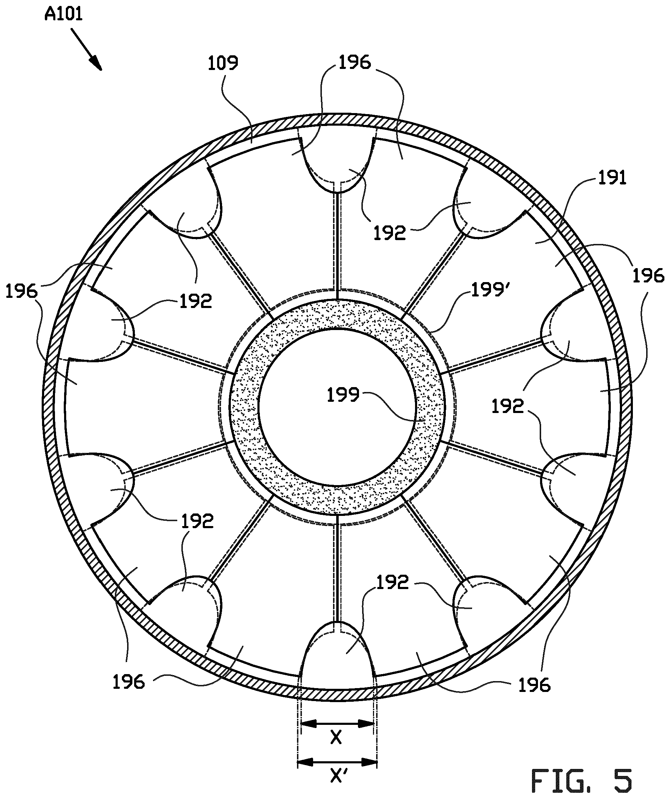

As shown in figures 4 and 5, in this example, the first guide body 191 and the second guide body 193 are each formed by a plurality of segments 196, 197 distributed circumferentially and radially expandable, wherein the one or more passageways 192 and/or the one or more singulating chambers 194 are formed at the transition between said segments 196, 197. Consequently, when the segments 196, 197 are expanded radially, the one or more passageways 192 and/or the one or more singulating chambers 194 formed between them are expanded in size.

The radial expansion of the segments 196, 197 may be varied automatically with an actuator element 199 that responds to the variations in the value V of the parameter P in the same way or in a similar manner as the identical first discrete medicaments M1. In particular, as shown in figure 5, the actuator element 199 may be centrally positioned between the segments 196 and it may comprise hygroscopically expandable material that, when subjected to a relatively high humidity, expands (see the expanded actuator element 199' in dashed lines) and drives the segments 196 radially outward, thereby increasing the dimensions of the supply passageways 192 defined by said segments 196.

Alternatively, the mechanical adjustment may be controlled automatically by suitable actuators and/or performed manually by the pharmacist based on instructions generated by the control unit 6, for example, displayed via one of the human machine interfaces 81, 82.

**LIST OF REFERENCE NUMERALS**

| | |
|---|---|
| 1 | pharmacy |
| 2 | dispensing environment |
| 21 | dispensing device |
| 22 | exchange station |
| 23 | exchange drawer |
| 3 | storage environment |
| 31-36 | stock containers |
| 4 | filling environment |
| 41 | filling station |
| 5 | external environment |
| 6 | control unit |
| 61 | processor |
| 62 | memory |
| 71 | first sensor |
| 72 | second sensor |
| 73 | third sensor |
| 74 | fourth sensor |
| 75 | weather sensor |
| 76 | meteorological database |
| 81 | human machine interface |
| 82 | human machine interface |
| 9, 9' | dispensing mechanism |
| 90, 90' | dispensing path |
| 91, 91' | guide body (supply body) |
| 92, 92' | supply passageway |
| 93, 93' | guide body (singulating body) |
| 94, 94' | singulating chamber |
| 95, 95' | guide body (dispensing body) |
| 96 | waiting chamber |
| 97 | outlet |
| 98 | circumferential recess |
| 99 | separation member |
| 109 | alternative dispensing mechanism |
| 190 | dispensing path |
| 191 | guide body (supply body) |
| 192 | supply passageway |
| 193 | guide body (singulating body) |
| 194 | singulating chamber |
| 195 | guide body (dispensing body) |
| 196 | segment |
| 197 | Segment |
| 198 | circumferential recess |
| 199 | actuator element |
| 199' | expanded actuator element |
| A1 | first medication dispensing container |
| A2 | second medication dispensing container |
| A101 | adjustable medication dispensing container |
| B1 | alternative first medication dispensing container |
| B2 | alternative second medication dispensing container |
| C | chosen medication dispensing container |
| D | calendar time |
| H1 | absolute humidity |
| H2 | relative humidity |
| M | medicament column |
| M1 | first discrete medicament |
| M2 | second discrete medicament |
| P | parameter |
| R1 | first range |
| R2 | second range |
| S1 | step "start of the method" |
| S2 | step "obtaining signals" |
| S3 | step "determining the value of the parameter" |
| S4 | step "is the value within a first range or a second range?" |
| S5 | step "has the range has changed with respect to the range determined in the previous cycle of the method?" |
| S6 | step "input of the medication dispensing |
| S7 | container chosen based on the range" step "output of an instruction to the human machine interface" |
| T | temperature |
| V | Value |
| W | meteorological data |
| X, X' | first dimension |
| Y, Y' | second dimension |
| Z, Z' | third dimension |

## Claims

1. Pharmacy (1) comprising a dispensing device (21) for dispensing discrete medicaments (M1, M2), a set of a first medication dispensing container (A1) and a second medication dispensing container (A2) having dimensions (X', Y') different from the first medication dispensing container (A1), at least one sensor (71-75), wherein the pharmacy (1) further comprises a control unit (6) that is configured for:
- obtaining a value (V) of a parameter (P) indicative of a climatological condition or variations in size and/or volume of the discrete medicaments (M1, M2) as a result of said climatological condition, wherein the climatological condition relates to humidity and/or temperature in an environment in which the discrete medicaments (M1, M2) are stored, filled, or dispensed (2, 3, 4), wherein the parameter is absolute humidity (H1) or relative humidity (H2); and wherein the value (V) of the parameter (P) is obtained by one of the following: measuring, by a sensor (71-75), the absolute humidity (H1) directly, measuring, by a sensor (71-75), the realtive humidity (H2) directly and calculating the absolute humidity (H1) based on the measured value (V) of relative humidity (H2) and a temperature (T) associated with said relative humidity (H2), or basing on or deriving from meteorological data (W) from a meteorological database (76) connectable to the control unit (6);
- determining if the value (V) of the parameter (P) is within a first range (R1) or a second range (R2), different from the first range (R1); and
- choosing, for identical discrete medicaments (M1) that are subject to hygroscopic expansion, between using the first medication dispensing container (A1) when the value (V) of the parameter (P) is within the first range (R1), and using the second medication dispensing container (A2) when the value (V) of the parameter (P) is within the second range (R2), to dispense the identical discrete medicaments (M1, wherein the control unit (6), after the chosen medication dispensing container (A2) of the first medication dispensing container (A1) and the second medication dispensing container (A2) has been filled with the identical discrete medicaments (M1), is configured for:
- controlling the dispensing device (21) to dispense the identical discrete medicaments (M1) with the use of the chosen medication dispensing container (A2).

2. Pharmacy (1) according to claim 1, wherein the at least one sensor comprises a first sensor (71, 72) for measuring the value (V) of the parameter (P) in a dispensing environment (2) in which the identical discrete medicaments (M1) are dispensed, and/or a second sensor (73, 74) connectable to the control unit, the second sensor for measuring the value (V) of the parameter (P) in a storage environment (3) and/or a filling environment (4) in which the identical discrete medicaments (M1) are stored or filled, respectively.

3. Pharmacy (1) according to claim 1, wherein the control unit (6) is configured for: determining a calendar time (D), wherein the value (V) of the parameter (P) is the calendar time (D), in particular a calendar date, a calendar month, a calendar quarter, a calendar season or another calendar-related period of time, and/or determining a time spent by the identical discrete medicaments (M1, M2) in the environment in which the discrete medicaments (M1, M2) were stored, filled, or dispensed (2, 3, 4).

4. Pharmacy (1) according to any one of claims 1-3, wherein the control unit (6) comprises a memory (62), wherein the control unit (6) is configured for identifying the first medication dispensing container (A1) and the second medication dispensing container (A2) as candidates for the choice (C) to be made, and for allocating in the memory (62) said first medication dispensing container (A1) and said second medication dispensing container (A2) for the identical discrete medicaments (M1).

5. Pharmacy (1) according to any one of claims 1-4, wherein the control unit (6) is operationally connected to a human machine interface (81, 82) for displaying the choice (C) as an instruction to an operator to fill the chosen medication dispensing container (A2) with the identical discrete medicaments (M1).

6. Pharmacy (1) according to any one of claims 1-5, wherein the first medication dispensing container (A1) and the second medication dispensing container (A2) each comprise a dispensing mechanism (9, 9') with one or more guide bodies (91, 93, 98; 91', 93', 98') that define dimensions (X, X'; Y, Y'; Z, Z') of at least a part of a dispensing path (90, 90') of the identical discrete medicaments (M1) through said dispensing mechanism (9, 9'), wherein the dimensions (X', Y', Z') of the dispensing path (90') defined by said one or more guide bodies (91', 93', 98') of the second medication dispensing container (A2) are different from the dimensions (X, Y, Z) of the dispensing path (90) defined by said one or more guide bodies (91, 93, 98) of the first medication dispensing container (A1), preferably wherein the dimensions (X, X'; Y, Y'; Z, Z') of the dispensing path (90, 90') defined by said one or more guide bodies (91, 93, 98; 91', 93', 98') are fixed for the first medication dispensing container (A1) and the second medication dispensing container (A2).

7. Pharmacy (1) comprising a dispensing device (21) for dispensing discrete medicaments (M1), wherein the pharmacy (1) comprises an adjustable medication dispensing container (A101) that is adjustable between a first configuration state with first dimensions (X, Y, Z) and a second configuration state with second dimensions (X', Y', Z'), different from the first dimensions (X, Y, Z), and at least one sensor (71-75), wherein the pharmacy (1) further comprises a control unit (6) that is configured for:
- obtaining a value (V) of a parameter (P) indicative of a climatological condition or variations in size and/or volumeof the discrete medicaments (M1, M2) as a result of said climatological condition, wherein the climatological condition relates to humidity and/or temperature in an environment in which the discrete medicaments (M1, M2) are stored, filled, or dispensed (2, 3, 4), wherein the parameter is absolute humidity (H1) or relative humidity (H1); and wherein the value (V) of the parameter (P) is obtained by one of the following: measuring, by a sensor (71-75), the absolute humidity (H1) directly, measuring, by a sensor (71-75), the realtive humidity (H2) directly and calculating the absolute humidity (H1) based on the measured value (V) of relative humidity (H2) and a temperature (T) associated with said relative humidity (H2), or basing on or deriving from meteorological data (W) from a meteorological database (76) connectable to the control unit (6);
- determining if the value (V) of the parameter (P) is within a first range (R1) or a second range (R2), different from the first range (R1); and
- generating an instruction for adjusting the adjustable medication dispensing container (A101), for identical discrete medicaments (M1) that are subject to hygroscopic expansion, wherein the adjustable medication dispensing container (A101) is adjusted to the first configuration state when the value (V) of the parameter (P) is within the first range (R1) and adjusted to the second configuration state when the value (V) of the parameter (P) is within the second range (R2), wherein the control unit (6), after the adjustment of the adjustable medication dispensing container (A101), is configured for:
- controlling the dispensing device (21) to dispense the identical discrete medicaments (M1) with the use of the adjustable medication dispensing container (A101).

8. Pharmacy (1) according to claim 7, wherein the adjustable medication dispensing container (A101) comprises a dispensing mechanism (109) with one or more guide bodies (191, 193, 198) that define dimensions (X, X'; Y, Y'; Z, Z') of at least a part of a dispensing path (190) of the identical discrete medicaments (M1) through said dispensing mechanism (109), wherein the one or more guide bodies (191, 193, 198) are mechanically adjustable to vary the dimensions (X, X'; Y, Y'; Z, Z') of the dispensing path (190) defined by said one or more guide bodies (191, 193, 198) between the first configuration state and the second configuration state.

9. Method for dispensing discrete medicaments (M1, M2), wherein the method comprises the steps of:
- obtaining, by a control unit (6) connectable to at least one sensor (71-75), a value (V) of a parameter (P) indicative of a climatological condition or variations in size and/or volume of the discrete medicaments (M1, M2) as a result of said climatological condition, wherein the climatological condition relates to humidity and/or temperature in an environment in which the discrete medicaments (M1, M2) are stored, filled, or dispensed (2, 3, 4), wherein the parameter is absolute humidity (H1) or relative humidity (H1); and wherein the value (V) of the parameter (P) is obtained by one of the following: measuring, by a sensor (71-75), the absolute humidity (H1) directly, measuring, by a sensor (71-75), the realtive humidity (H2) directly and calculating the absolute humidity (H1) based on the measured value (V) of relative humidity (H2) and a temperature (T) associated with said relative humidity (H2), or basing on or deriving from meteorological data (W) from a meteorological database (76)connectable to the control unit (6);
- determining, by the control unit (6), if the value (V) of the parameter (P) is within a first range (R1) or a second range (R2), different from the first range (R1); and
- choosing, by the control unit (6), for identical discrete medicaments (M1) that are subject to hygroscopic expansion, between using a first medication dispensing container (A1) when the value (V) of the parameter (P) is within the first range (R1), and using a second medication dispensing container (A2) when the value (V) of the parameter (P) is within the second range (R2), to dispense the identical discrete medicaments (M1), wherein the second medication dispensing container (A2) has different dimensions than the first medication dispensing container (A1); - filling a chosen medication dispensing container (A2) of the first medication dispensing container (A1) and the second medication dispensing container (A2) with the identical discrete medicaments (M1); and - dispensing, by a dispensing device (21), the identical discrete medicaments (M1) with the use of the chosen medication dispensing container (A2).

10. Method according to claim 9, wherein the value (V) of the parameter (P) is measured, by the at least one sensor (72), in a dispensing environment (2) in which the identical discrete medicaments (M1) are dispensed; and/or wherein the value (V) of the parameter (P) is measured, by the at least one sensor (73, 74), in a storage environment (3) and/or a filling environment (4) in which the identical discrete medicaments (M1) are stored and/or filled, respectively.

11. Method according to claim 9, wherein the value (V) of the parameter (P) is a calendar time (D), in particular a calendar date, a calendar month, a calendar quarter or another calendar-related period of time; and/or wherein the value (V) of the parameter (P) is time spent by the identical discrete medicaments (M1, M2) in the environment in which the discrete medicaments (M1, M2) are stored, filled, or dispensed (2, 3, 4).

12. Method according to any one of the claims 9-11, wherein the method comprises the step of assigning, by the control unit (6), the first medication dispensing container (A1) and the second medication dispensing container (A2) to the identical discrete medicaments (M1), prior to the step of choosing between the first medication dispensing container (A1) and the second medication dispensing container (A2).

13. Method according to any one of the claims 9-12, wherein the method further comprises the step of displaying, by a human-machine interface (81, 82), the choice (C) made in the step of choosing between the first medication dispensing container (A1) and the second medication dispensing container (A2), preferably wherein the displayed choice (C) is an instruction to an operator to fill the chosen medication dispensing container (A2) with the identical discrete medicaments (M1).

14. Method according to any one of the claims 9-13, wherein the first medication dispensing container (A1) and the second medication dispensing container (A2) each comprise a dispensing mechanism (9, 9') with one or more guide bodies (91, 93, 95; 91', 93', 95') that define dimensions (X, X'; Y, Y'; Z, Z') of at least a part of a dispensing path (90, 90') of the identical discrete medicaments (M1) through said dispensing mechanism (9, 9'), wherein the dimensions (X', Y', Z') of the dispensing path (90') defined by said one or more guide bodies (91', 93', 95') of the second medication dispensing container (A2) are different from the dimensions (X, Y, Z) of the dispensing path (90) defined by said one or more guide bodies (91, 93, 95) of the first medication dispensing container (A1), preferably wherein the dimensions of the dispensing path (90, 90') defined by said one or more guide bodies (91, 93, 95; 91', 93', 95') are fixed for the first medication dispensing container (A1) and the second medication dispensing container (A2).

15. Method for dispensing discrete medicaments (M1, M2), wherein the method comprises the steps of:
- obtaining, by a control unit (6), a value (V) of a parameter (P) indicative of a climatological condition or variations in size and/or volume of the discrete medicaments (M1, M2) as a result of said climatological condition, wherein the climatological condition relates to humidity and/or temperature in an environment in which the discrete medicaments (M1, M2) are stored, filled, or dispensed (2, 3, 4), wherein the parameter is absolute humidity (H1) or relative humidity (H1); and wherein the value (V) of the parameter (P) is obtained by one of the following: measuring, by a sensor (71-75), the absolute humidity (H1) directly, measuring, by a sensor (71-75), the realtive humidity (H2) directly and calculating the absolute humidity (H1) based on the measured value (V) of relative humidity (H2) and a temperature (T) associated with said relative humidity (H2), or basing on or deriving from meteorological data (W) from a meteorological database (76)connectable to the control unit (6);
- determining, by the control unit (6), if the value (V) of the parameter (P) is within a first range (R1) or a second range (R2), different from the first range (R1);
- generating, by the control unit (6), an instruction for adjusting dimensions (X, X'; Y, Y'; Z, Z') of an adjustable medication dispensing container (A101), for identical discrete medicaments (M1) that are subject to hygroscopic expansion, wherein the adjustable medication dispensing container (A101) is adjusted to a first configuration state with first dimensions (X, Y, Z) when the value (V) of the parameter (P) is within the first range (R1) and adjusted to a second configuration state with second dimensions (X', Y', Z'), different from the first dimensions (X, Y, Z), when the value (V) of the parameter (P) is within the second range (R2);
- dispensing, by a dispensing device (21), the identical discrete medicaments (M1) with the use of the adjustable medication dispensing container (A101).

16. Method according to claim 15, wherein the adjustable medication dispensing container (A101) comprises a dispensing mechanism (109) with one or more guide bodies (191, 193, 198) that define dimensions of at least a part of a dispensing path (190) of the identical discrete medicaments (M1) through said dispensing mechanism (109), wherein the one or more guide bodies (191, 193, 198) are mechanically adjustable to vary the dimensions (X, X'; Y, Y'; Z, Z') of the dispensing path (190) defined by said one or more guide bodies (191, 193, 198) between the first configuration state and the second configuration state; and/or wherein the adjustable medication dispensing container (A101) comprises an actuator element (199) for mechanically adjusting the one or more guide bodies (191, 193, 198), wherein the actuator element (199) comprises hygroscopically expandable material.

17. Medication dispensing container (A101) for use in a pharmacy (1), wherein the medication dispensing container (A101) comprises a dispensing mechanism (109) with one or more guide bodies (191, 193, 198) which are mechanically adjustable between a first configuration state with first dimensions (X, Y, Z) and a second configuration state with second dimensions (X', Y', Z'), different from the first dimensions (X, Y, Z), wherein the medication dispensing container (A101) comprises an actuator element (199) for mechanically adjusting the one or more guide bodies (191, 193, 198), wherein the actuator element (199) comprises hygroscopically expandable material.

18. Computer program product comprising a non-transitory computer-readable medium (62) holding instructions that, when executed by a processor (61), cause a pharmacy (1, 100) according to any one of claims 1-8 to perform the steps of the method according to any one of claims 9-17.

## Patentansprüche

1. Apotheke (1), umfassend eine Ausgabeeinrichtung (21) zum Ausgeben diskreter Arzneimittel (M1, M2), einen Satz aus einem ersten Arzneimittelausgabebehälter (A1) und einem zweiten Arzneimittelausgabebehälter (A2) mit Abmessungen (X', Y'), die sich von denen des ersten Arzneimittelausgabebehälters (A1) unterscheiden, mindestens einen Sensor (71-75), wobei die Apotheke (1) ferner eine Steuereinheit (6) umfasst, die dazu konfiguriert ist:
- einen Wert (V) eines Parameters (P) zu erhalten, der eine klimatischen Bedingung oder Größen- und/oder Volumenänderungen der diskreten Arzneimittel (M1, M2) infolge der klimatischen Bedingung anzeigt, wobei sich die klimatischen Bedingung auf die Luftfeuchtigkeit und/oder die Temperatur in einer Umgebung bezieht, in der die diskreten Arzneimittel (M1, M2) gelagert, abgefüllt oder ausgegeben werden (2, 3, 4), wobei der Parameter die absolute Luftfeuchtigkeit (H1) oder die relative Luftfeuchtigkeit (H2) ist; und wobei der Wert (V) des Parameters (P) erhalten wird durch eines von Folgendem: direktes Messen der absoluten Luftfeuchtigkeit (H1) mittels eines Sensors (71-75), direktes Messen der relativen Luftfeuchtigkeit (H2) mittels eines Sensors (71-75) und Berechnen der absoluten Luftfeuchtigkeit (H1) basierend auf dem gemessenen Wert (V) der relativen Luftfeuchtigkeit (H2) und einer mit der relativen Luftfeuchtigkeit (H2) assoziierten Temperatur (T), oder basierend auf oder ableitend aus meteorologischen Daten (W) von einer mit der Steuereinheit (6) verbindbaren meteorologischen Datenbank (76);
- zu bestimmen, ob der Wert (V) des Parameters (P) innerhalb eines ersten Bereichs (R1) oder eines zweiten Bereichs (R2), der sich vom ersten Bereich (R1) unterscheidet, liegt; und
- für identische diskrete Medikamente (M1), die hygroskopischer Ausdehnung unterliegen, auszuwählen zwischen einer Nutzung des ersten Arzneimittelausgabebehälters (A1), wenn der Wert (V) des Parameters (P) innerhalb des ersten Bereichs (R1) liegt, und Nutzung des zweiten Arzneimittelausgabebehälters (A2), wenn der Wert (V) des Parameters (P) innerhalb des zweiten Bereichs (R2) liegt, um die identischen diskreten Arzneimittel (M1) auszugeben, wobei die Steuereinheit (6) dazu konfiguriert ist, nachdem der ausgewählte Arzneimittelausgabebehälter (A2) von dem ersten Arzneimittelausgabebehälter (A1) und dem zweiten Arzneimittelausgabebehälter (A2) mit den identischen diskreten Arzneimitteln (M1) befüllt worden ist:
- die Ausgabeeinrichtung (21) zu steuern, um die identischen diskreten Arzneimittel (M1) unter Verwendung des ausgewählten Arzneimittelausgabebehälters (A2) auszugeben.

2. Apotheke (1) gemäß Anspruch 1, wobei der mindestens eine Sensor, einen ersten Sensor (71, 72) zur Messung des Wertes (V) des Parameters (P) in einer Ausgabeumgebung (2), in der die identischen diskreten Arzneimittel (M1) ausgegeben werden, und/oder einen mit der Steuereinheit verbindbaren zweiten Sensor (73, 74) zur Messung des Wertes (V) des Parameters (P) in einer Lagerumgebung (3) und/oder einer Abfüllumgebung (4), in der die identischen diskreten Arzneimittel (M1) entsprechend gelagert oder abgefüllt werden, umfasst.

3. Apotheke (1) gemäß Anspruch 1, wobei die Steuereinheit (6) dazu konfiguriert ist: eine Kalenderzeit (D) zu bestimmen, wobei der Wert (V) des Parameters (P) die Kalenderzeit (D) ist, insbesondere ein Kalenderdatum, ein Kalendermonat, ein Kalenderquartal, eine Jahreszeit oder ein anderer kalenderbezogener Zeitraum; und/oder eine Verweildauer der identischen diskreten Arzneimittel (M1, M2) in der Umgebung zu bestimmen, in der die diskreten Arzneimittel (M1, M2) gelagert, abgefüllt oder ausgegeben wurden (2, 3, 4).

4. Apotheke (1) gemäß einem der Ansprüche 1 bis 3, wobei die Steuereinheit (6) einen Speicher (62) umfasst, wobei die Steuereinheit (6) dazu konfiguriert ist, den ersten Arzneimittelausgabebehälter (A1) und den zweiten Arzneimittelausgabebehälter (A2) als Kandidaten für die zu treffende Auswahl (C) zu identifizieren und im Speicher (62) den genannten ersten Arzneimittelausgabebehälter (A1) und den genannten zweiten Arzneimittelausgabebehälter (A2) für die identischen diskreten Arzneimittel (M1) zuzuordnen.

5. Apotheke (1) gemäß einem der Ansprüche 1 bis 4, wobei die Steuereinheit (6) mit einer Mensch-Maschine-Schnittstelle (81, 82) operativ verbunden ist, um die Auswahl (C) als eine Anweisung an eine Bedienperson anzuzeigen, den ausgewählten Arzneimittelausgabebehälter (A2) mit den identischen diskreten Arzneimitteln (M1) zu befüllen.

6. Apotheke (1) gemäß einem der Ansprüche 1 bis 5, wobei der erste Arzneimittelausgabebehälter (A1) und der zweite Arzneimittelausgabebehälter (A2) jeweils einen Ausgabemechanismus (9, 9') mit einem oder mehreren Führungskörpern (91, 93, 98; 91', 93', 98') umfassen, die Abmessungen (X, X'; Y, Y'; Z, Z') von zumindest einem Teil eines Ausgabeweges (90, 90') der identischen diskreten Arzneimittel (M1) durch den Ausgabemechanismus (9, 9') definieren, wobei sich die Abmessungen (X', Y', Z') des durch die Führungskörper (91', 93', 98') des zweiten Arzneimittelausgabebehälters (A2) definierten Ausgabeweges (90') von den Abmessungen (X, Y, Z) des durch die Führungskörper (91, 93, 98) des ersten Arzneimittelausgabebehälters (A1) definierten Ausgabeweges (90) unterscheiden, wobei vorzugsweise die Abmessungen (X, X'; Y, Y'; Z, Z') des durch den einen oder die mehreren Führungskörper (91, 93, 98; 91', 93', 98') definierten Ausgabeweges (90, 90') sowohl für den ersten Arzneimittelausgabebehälter (A1) als auch für den zweiten Arzneimittelausgabebehälter (A2) festgelegt sind.

7. Apotheke (1), umfassend eine Ausgabeeinrichtung (21) zum Ausgeben diskreter Arzneimittel (M1), wobei die Apotheke (1) einen einstellbaren Arzneimittelausgabebehälter (A101) umfasst, der zwischen einem ersten Konfigurationszustand mit ersten Abmessungen (X, Y, Z) und einem zweiten Konfigurationszustand mit zweiten Abmessungen (X', Y', Z'), die sich von den ersten Abmessungen (X, Y, Z) unterscheiden, einstellbar ist, und mindestens einen Sensor (71-75), wobei die Apotheke (1) ferner eine Steuereinheit (6) umfasst, die dazu konfiguriert ist:
- einen Wert (V) eines Parameters (P) zu erhalten, der eine klimatischen Bedingung oder Größen- und/oder Volumenänderungen der diskreten Arzneimittel (M1, M2) infolge der klimatischen Bedingung anzeigt, wobei sich die klimatischen Bedingung auf die Luftfeuchtigkeit und/oder die Temperatur in einer Umgebung bezieht, in der die diskreten Arzneimittel (M1, M2) gelagert, abgefüllt oder ausgegeben werden (2, 3, 4), wobei der Parameter die absolute Luftfeuchtigkeit (H1) oder die relative Luftfeuchtigkeit (H2) ist; und wobei der Wert (V) des Parameters (P) erhalten wird durch eines von Folgendem: direktes Messen der absoluten Luftfeuchtigkeit (H1) mittels eines Sensors (71-75), direktes Messen der relativen Luftfeuchtigkeit (H2) mittels eines Sensors (71-75) und Berechnen der absoluten Luftfeuchtigkeit (H1) basierend auf dem gemessenen Wert (V) der relativen Luftfeuchtigkeit (H2) und einer mit der relativen Luftfeuchtigkeit (H2) assoziierten Temperatur (T), oder basierend auf oder ableitend aus meteorologischen Daten (W) von einer mit der Steuereinheit (6) verbindbaren meteorologischen Datenbank (76);
- zu bestimmen, ob der Wert (V) des Parameters (P) innerhalb eines ersten Bereichs (R1) oder eines zweiten Bereichs (R2), der sich vom ersten Bereich (R1) unterscheidet, liegt; und
- eine Anweisung zum Einstellen des einstellbaren Arzneimittelausgabebehälters (A101) zu erzeugen für identische diskrete Arzneimittel (M1), die hygroskopischer Ausdehnung unterliegen, wobei der einstellbare Arzneimittelausgabebehälter (A101) in den ersten Konfigurationszustand eingestellt wird, wenn der Wert (V) des Parameters (P) innerhalb des ersten Bereichs (R1) liegt, und in den zweiten Konfigurationszustand eingestellt wird, wenn der Wert (V) des Parameters (P) innerhalb des zweiten Bereichs (R2) liegt, wobei die Steuereinheit (6) dazu konfiguriert ist, nach dem Einstellen des einstellbaren Arzneimittelausgabebehälters (A101):
- die Ausgabeeinrichtung (21) zu steuern, um die identischen diskreten Arzneimittel (M1) unter Verwendung des einstellbaren Arzneimittelausgabebehälters (A101) auszugeben.

8. Apotheke (1) gemäß Anspruch 7, wobei der einstellbare Arzneimittelausgabebehälter (A101) einen Ausgabemechanismus (109) mit einem oder mehreren Führungskörpern (191, 193, 198) umfasst, die Abmessungen (X, X'; Y, Y'; Z, Z') von zumindest einem Teil eines Ausgabeweges (190) der identischen diskreten Arzneimittel (M1) durch den Ausgabemechanismus (109) definieren, wobei der eine oder die mehreren Führungskörper (191, 193, 198) mechanisch einstellbar sind, um die durch den einen oder die mehreren Führungskörper (191, 193, 198) definierten Abmessungen (X, X'; Y, Y'; Z, Z') des Ausgabeweges (190) zwischen dem ersten Konfigurationszustand und dem zweiten Konfigurationszustand zu variieren.

9. Verfahren zum Ausgeben diskreter Arzneimittel (M1, M2), wobei das Verfahren die Schritte umfasst:
- Erhalten eines Wertes (V) eines Parameters (P), der eine klimatischen Bedingung oder Größen- und/oder Volumenänderungen der diskreten Arzneimittel (M1, M2) infolge der klimatischen Bedingung anzeigt, durch eine mit mindestens einem Sensor (71-75) verbindbare Steuereinheit (6), wobei sich die klimatischen Bedingung auf die Luftfeuchtigkeit und/oder die Temperatur in einer Umgebung bezieht, in der die diskreten Arzneimittel (M1, M2) gelagert, abgefüllt oder ausgegeben werden (2, 3, 4), wobei der Parameter die absolute Luftfeuchtigkeit (H1) oder die relative Luftfeuchtigkeit (H2) ist; und wobei der Wert (V) des Parameters (P) erhalten wird durch eines von Folgendem: direktes Messen der absoluten Luftfeuchtigkeit (H1) mittels eines Sensors (71-75), direktes Messen der relativen Luftfeuchtigkeit (H2) mittels eines Sensors (71-75) und Berechnen der absoluten Luftfeuchtigkeit (H1) basierend auf dem gemessenen Wert (V) der relativen Luftfeuchtigkeit (H2) und einer mit der relativen Luftfeuchtigkeit (H2) assoziierten Temperatur (T), oder basierend auf oder ableitend aus meteorologischen Daten (W) von einer mit der Steuereinheit (6) verbindbaren meteorologischen Datenbank (76);
- Bestimmen durch die Steuereinheit (6), ob der Wert (V) des Parameters (P) innerhalb eines ersten Bereichs (R1) oder eines zweiten Bereichs (R2), der sich vom ersten Bereich (R1) unterscheidet, liegt;
- Auswählen durch die Steuereinheit (6) für identische diskrete Arzneimittel (M1), die hygroskopischer Ausdehnung unterliegen, zwischen der Nutzung eines ersten Arzneimittelausgabebehälter (A1), wenn der Wert (V) des Parameters (P) innerhalb des ersten Bereichs (R1) liegt, und der Nutzung des zweiten Arzneimittelausgabebehälter (A2), wenn der Wert (V) des Parameters (P) innerhalb des zweiten Bereichs (R2) liegt, um die identisch diskreten Medikamente auszugeben, wobei der zweite Arzneimittelausgabebehälter (A2) andere Abmessungen als der erste Arzneimittelausgabebehälter (A1) aufweist;
- Befüllen eines ausgewählten Arzneimittelausgabebehälters von dem ersten Arzneimittelausgabebehälter (A1) und dem zweiten Arzneimittelausgabebehälter (A2) mit den identischen diskreten Arzneimitteln (M1); und
- Ausgeben der identischen diskreten Arzneimittel (M1) mittels einer Ausgabeeinrichtung (21) unter Verwendung des ausgewählten Arzneimittelausgabebehälters (A2).

10. Verfahren gemäß Anspruch 9, wobei der Wert (V) des Parameters (P) durch den mindestens einen Sensor (72) in einer Ausgabenumgebung (2), in der die identischen diskreten Arzneimittel (M1) ausgegeben werden, gemessen wird; und/oder wobei der Wert (V) des Parameters (P) durch den mindestens einen Sensor (73, 74) in einer Lagerumgebung (3) und/oder einer Abfüllumgebung (4) gemessen wird, in der die identischen diskreten Arzneimittel (M1) entsprechend gelagert und/oder abgefüllt werden.

11. Verfahren gemäß Anspruch 9, wobei der Wert (V) des Parameters (P) eine Kalenderzeit (D) ist, insbesondere ein Kalenderdatum, ein Kalendermonat, ein Kalenderquartal oder ein anderer kalenderbezogener Zeitraum; und/oder wobei der Wert (V) des Parameters (P) die Verweildauer der identischen diskreten Arzneimittel (M1, M2) in der Umgebung ist, in der die diskreten Arzneimittel (M1, M2) gelagert, abgefüllt oder ausgegeben werden (2, 3, 4).

12. Verfahren gemäß einem der Ansprüche 9 bis 11, wobei das Verfahren den Schritt umfasst, durch die Steuereinheit (6) den ersten Arzneimittelausgabebehälter (A1) und den zweiten Arzneimittelausgabebehälter (A2) den identischen diskreten Arzneimitteln (M1) zuzuordnen, vor dem Schritt des Auswählens zwischen dem ersten Arzneimittelausgabebehälter (A1) und dem zweiten Arzneimittelausgabebehälter (A2).

13. Verfahren gemäß einem der Ansprüche 9 bis 12, wobei das Verfahren ferner den Schritt umfasst, durch eine Mensch-Maschine-Schnittstelle (81, 82) die getroffene Auswahl (C), die in dem Schritt des Auswählens zwischen dem ersten Arzneimittelausgabebehälter (A1) und dem zweiten Arzneimittelausgabebehälter (A2) gemacht wurde, anzuzeigen, wobei vorzugsweise die angezeigte Auswahl (C) eine Anweisung an eine Bedienperson ist, den ausgewählten Arzneimittelausgabebehälter (A2) mit den identischen diskreten Arzneimitteln (M1) zu befüllen.

14. Verfahren gemäß einem der Ansprüche 9 bis 13, wobei der erste Arzneimittelausgabebehälter (A1) und der zweite Arzneimittelausgabebehälter (A2) jeweils einen Ausgabemechanismus (9, 9') mit einem oder mehreren Führungskörpern (91, 93, 95; 91', 93', 95') umfassen, die Abmessungen (X, X'; Y, Y'; Z, Z') von zumindest einem Teil eines Ausgabeweges (90, 90') der identischen diskreten Arzneimittel (M1) durch den Ausgabemechanismus (9, 9') definieren, wobei sich die Abmessungen (X', Y', Z') des durch den einen oder die mehreren Führungskörper (91', 93', 95') des zweiten Arzneimittelausgabebehälters (A2) definierten Ausgabeweges (90') von den Abmessungen (X, Y, Z) des durch den einen oder die mehreren Führungskörper (91, 93, 95) des ersten Arzneimittelausgabebehälters (A1) definierten Ausgabeweges (90) unterscheiden, wobei vorzugsweise die Abmessungen des durch den einen oder die mehreren Führungskörper (91, 93, 95; 91', 93', 95') definierten Ausgabeweges (90, 90') sowohl für den ersten Arzneimittelausgabebehälter (A1) als auch den zweiten Arzneimittelausgabebehälter (A2) festgelegt sind.

15. Verfahren zum Ausgeben diskreter Arzneimittel (M1, M2), wobei das Verfahren die Schritte umfasst:
- Erhalten eines Werts (V) eines Parameters (P), der eine klimatische Bedingung oder Größen- und/oder Volumenänderungen der diskreten Arzneimittel (M1, M2) infolge der klimatischen Bedingung anzeigt, durch eine Steuereinheit (6), wobei sich die klimatische Bedingung auf die Luftfeuchtigkeit und/oder die Temperatur in einer Umgebung bezieht, in der die diskreten Arzneimittel (M1, M2) gelagert, abgefüllt oder ausgegeben werden (2, 3, 4), wobei der Parameter die absolute Luftfeuchtigkeit (H1) oder die relative Luftfeuchtigkeit (H2) ist; und wobei der Wert (V) des Parameters (P) erhalten wird durch eines von Folgendem: direktes Messen der absoluten Luftfeuchtigkeit (H1) mittels eines Sensors (71-75), direktes Messen der relativen Luftfeuchtigkeit (H2) mittels eines Sensors (71-75) und Berechnen der absoluten Luftfeuchtigkeit (H1) basierend auf dem gemessenen Wert (V) der relativen Luftfeuchtigkeit (H2) und einer mit der relativen Luftfeuchtigkeit (H2) assoziierten Temperatur (T), oder basierend auf oder ableitend aus meteorologischen Daten (W) von einer mit der Steuereinheit (6) verbindbaren meteorologischen Datenbank (76);
Bestimmen durch die Steuereinheit (6), ob der Wert (V) des Parameters (P) innerhalb eines ersten Bereichs (R1) oder eines zweiten Bereichs (R2), der sich vom ersten Bereich (R1) unterscheidet, liegt;
Erzeugen einer Anweisung durch die Steuereinheit (6) zum Einstellen von Abmessungen (X, X'; Y, Y'; Z, Z') eines einstellbaren Arzneimittelausgabebehälters (A101) für identische diskrete Arzneimittel (M1), die einer hygroskopischen Ausdehnung unterliegen, wobei der einstellbare Arzneimittelausgabebehälter (A101) in einen ersten Konfigurationszustand mit ersten Abmessungen (X, Y, Z) eingestellt wird, wenn der Wert (V) des Parameters (P) innerhalb des ersten Bereichs (R1) liegt, und in einen zweiten Konfigurationszustand mit zweiten Abmessungen (X', Y', Z'), die sich von den ersten Abmessungen (X, Y, Z) unterscheiden, eingestellt wird, wenn der Wert (V) des Parameters (P) innerhalb des zweiten Bereichs (R2) liegt;
Ausgeben der identischen diskreten Arzneimittel (M1) durch eine Ausgabeeinrichtung (21) unter Verwendung des einstellbaren Arzneimittelausgabebehälters (A101).

16. Verfahren gemäß Anspruch 15, wobei der einstellbare Arzneimittelausgabebehälter (A101) einen Ausgabemechanismus (109) mit einem oder mehreren Führungskörpern (191, 193, 198) umfasst, die Abmessungen zumindest eines Teils eines Ausgabeweges (190) der identischen diskreten Arzneimittel (M1) durch Ausgabemechanismus (109) definieren, wobei der eine oder die mehreren Führungskörper (191, 193, 198) mechanisch einstellbar sind, um die durch den einen oder die mehreren Führungskörper (191, 193, 198) definierten Abmessungen (X, X'; Y, Y'; Z, Z') des Ausgabeweges (190) zwischen dem ersten Konfigurationszustand und dem zweiten Konfigurationszustand zu variieren; und/oder wobei der einstellbare Arzneimittelausgabebehälter (A101) ein Aktuatorelement (199) zum mechanischen Einstellen des einen oder der mehreren Führungskörper (191, 193, 198) umfasst, wobei das Aktuatorelement (199) ein hygroskopisch expandierbares Material umfasst.

17. Medikamentenausgabebehälter (A101) zur Verwendung in einer Apotheke (1), wobei der Medikamentenausgabebehälter (A101) einen Ausgabemechanismus (109) mit einem oder mehreren Führungskörpern (191, 193, 198) umfasst, die zwischen einem ersten Konfigurationszustand mit ersten Abmessungen (X, Y, Z) und einem zweiten Konfigurationszustand mit zweiten Abmessungen (X', Y', Z'), die sich von den ersten Abmessungen (X, Y, Z) unterscheiden, mechanisch einstellbar sind, wobei der Medikamentenausgabebehälter (A101) ein Aktuatorelement (199) zum mechanischen Einstellen des einen oder der mehreren Führungselemente (191, 193, 198) umfasst, wobei das Aktuatorelement (199) hygroskopisch expandierbares Material umfasst.

18. Computerprogrammprodukt, umfassend ein nichtflüchtiges computerlesbares Medium (62), das Anweisungen enthält, die, wenn sie von einem Prozessor (61) ausgeführt werden, eine Apotheke (1, 100) gemäß einem der Ansprüche 1 bis 8 dazu veranlassen, die Schritte des Verfahrens gemäß einem der Ansprüche 9 bis 17 auszuführen.

## Revendications

1. Pharmacie (1) comprenant un dispositif de distribution (21) pour distribuer des médicaments discrets (M1, M2), un ensemble d'un premier récipient de distribution de médicaments (A1) et d'un deuxième récipient de distribution de médicaments (A2) ayant des dimensions (X', Y') différentes de celles du premier récipient de distribution de médicaments (A1), au moins un capteur (71-75), où la pharmacie (1) comprend en outre une unité de commande (6) qui est configurée pour :
- obtenir une valeur (V) d'un paramètre (P) indicatif d'une condition climatique ou de variations de taille et/ou de volume des médicaments discrets (M1, M2) résultant de ladite condition climatique, où la condition climatique concerne l'humidité et/ou la température dans un environnement dans lequel les médicaments discrets (M1, M2) sont stockés, remplis ou distribués (2, 3, 4), où le paramètre est l'humidité absolue (H1) ou l'humidité relative (H2) ;
et où la valeur (V) du paramètre (P) est obtenue par l'une des méthodes suivantes : mesurer, par un capteur (71-75), l'humidité absolue (H1) directement, mesurer, par un capteur (71-75), l'humidité relative (H2) directement et calculer l'humidité absolue (H1) sur la base de la valeur mesurée (V) de l'humidité relative (H2) et d'une température (T) associée à ladite humidité relative (H2), ou se baser sur ou dériver de données météorologiques (W) d'une base de données météorologique (76) pouvant être connectée à l'unité de commande (6) ;
- déterminer si la valeur (V) du paramètre (P) est comprise dans une première plage (R1) ou une deuxième plage (R2), différente de la première plage (R1) ; et
- choisir, pour des médicaments discrets (M1) identiques qui sont sujets à une expansion hygroscopique, entre l'utilisation du premier récipient de distribution de médicaments (A1) lorsque la valeur (V) du paramètre (P) est comprise dans la première plage (R1), et l'utilisation du deuxième récipient de distribution de médicaments (A2) lorsque la valeur (V) du paramètre (P) est comprise dans la deuxième plage (R2), afin de distribuer les médicaments discrets (M1) identiques, où l'unité de commande (6), après que le récipient de distribution de médicaments (A2) choisi parmi le premier récipient de distribution de médicaments (A1) et le deuxième récipient de distribution de médicaments (A2) a été rempli avec les médicaments discrets (M1) identiques, est configurée pour :
- commander au dispositif de distribution (21) de distribuer les médicaments discrets (M1) identiques à l'aide du récipient de distribution de médicaments (A2) choisi.

2. Pharmacie (1) selon la revendication 1, où l'au moins un capteur comprend un premier capteur (71, 72) pour mesurer la valeur (V) du paramètre (P) dans un environnement de distribution (2) dans lequel les médicaments discrets (M1) identiques sont distribués, et/ou un deuxième capteur (73, 74) pouvant être connecté à l'unité de commande, le deuxième capteur pour mesurer la valeur (V) du paramètre (P) dans un environnement de stockage (3) et/ou un environnement de remplissage (4) dans lequel les médicaments discrets (M1) identiques sont stockés ou remplis, respectivement .

3. Pharmacie (1) selon la revendication 1, où l'unité de commande (6) est configurée pour : déterminer un temps calendaire (D), où la valeur (V) du paramètre (P) est le temps calendaire (D), en particulier une date calendaire, un mois civil, un trimestre civil, une saison calendaire ou une autre période de temps liée au calendrier, et/ou déterminer un temps passé par les médicaments discrets identiques (M1, M2) dans l'environnement dans lequel les médicaments discrets (M1, M2) ont été stockés, remplis ou distribués (2, 3, 4).

4. Pharmacie (1) selon l'une quelconque des revendications 1 à 3, où l'unité de commande (6) comprend une mémoire (62), où l'unité de commande (6) est configurée pour identifier le premier récipient de distribution de médicaments (A1) et le deuxième récipient de distribution de médicaments (A2) en tant que candidats pour le choix (C) à effectuer, et pour attribuer dans la mémoire (62) ledit premier récipient de distribution de médicaments (AI) et ledit deuxième récipient de distribution de médicaments (A2) pour les médicaments discrets (M1) identiques.

5. Pharmacie (1) selon l'une quelconque des revendications 1 à 4, où l'unité de commande (6) est connectée de manière opérationnelle à une interface homme-machine (81, 82) pour afficher le choix (C) en tant qu'une instruction à un opérateur de remplir le récipient de distribution de médicaments (A2) choisi avec les médicaments discrets (M1) identiques.

6. Pharmacie (1) selon l'une quelconque des revendications 1 à 5, où le premier récipient de distribution de médicaments (A1) et le deuxième récipient de distribution de médicaments (A2) comprennent chacun un mécanisme de distribution (9, 9') avec un ou plusieurs corps de guidage (91, 93, 98 ; 91', 93', 98') qui définissent des dimensions (X, X' ; Y, Y' ; Z, Z') d'au moins une partie d'un trajet de distribution (90, 90') des médicaments discrets (M1) identiques à travers ledit mécanisme de distribution (9, 9'), où les dimensions (X', Y', Z') du trajet de distribution (90') défini par lesdits un ou plusieurs corps de guidage (91', 93', 98') du deuxième récipient de distribution de médicaments (A2) sont différentes des dimensions (X, Y, Z) du trajet de distribution (90) défini par lesdits un ou plusieurs corps de guidage (91, 93, 98) du premier récipient de distribution de médicaments (A1), de préférence, où les dimensions (X, X' ; Y, Y' ; Z, Z') du trajet de distribution (90, 90') défini par lesdits un ou plusieurs corps de guidage (91, 93, 98 ; 91', 93', 98') sont fixées pour le premier récipient de distribution de médicaments (A1) et le deuxième récipient de distribution de médicaments (A2).

7. Pharmacie (1) comprenant un dispositif de distribution (21) pour distribuer des médicaments discrets (M1), où la pharmacie (1) comprend un récipient de distribution de médicaments réglable (A101) qui est réglable entre un premier état de configuration avec des premières dimensions (X, Y, Z) et un deuxième état de configuration avec des deuxièmes dimensions (X', Y', Z'), différentes des premières dimensions (K, Y, Z), et au moins un capteur (71-75), où la pharmacie (1) comprend en outre une unité de commande (6) qui est configurée pour :
- obtenir une valeur (V) d'un paramètre (P) indicatif d'une condition climatique ou de variations de taille et/ou de volume des médicaments discrets (M1, M2) résultant de ladite condition climatique, où la condition climatique concerne l'humidité et/ou la température dans un environnement dans lequel les médicaments discrets (M1, M2) sont stockés, remplis ou distribués (2, 3, 4), où le paramètre est l'humidité absolue (H1) ou l'humidité relative (H2) ; et où la valeur (V) du paramètre (P) est obtenue par l'une des méthodes suivantes : mesurer, par un capteur (71-75), l'humidité absolue (H1) directement, mesurer, par un capteur (71-75), l'humidité relative (H2) directement et calculer l'humidité absolue (H1) sur la base de la valeur mesurée (V) de l'humidité relative (H2) et d'une température (T) associée à ladite humidité relative (H2), ou se baser sur ou dériver de données météorologiques (W) d'une base de données météorologique (76) pouvant être connectée à l'unité de commande (6) ;
- déterminer si la valeur (V) du paramètre (P) est comprise dans une première plage (R1) ou une deuxième plage (R2), différente de la première plage (R1) ; et
- générer une instruction de réglage du récipient de distribution de médicaments réglable (A101), pour des médicaments discrets (M1) identiques qui sont sujets à une expansion hygroscopique, où le récipient de distribution de médicaments réglable (A101) est réglé sur le premier état de configuration lorsque la valeur (V) du paramètre (P) est comprise dans la première plage (R1) et réglé sur le deuxième état de configuration lorsque la valeur (V) du paramètre (P) est comprise dans la deuxième plage (R2), où l'unité de commande (6), après le réglage du récipient de distribution de médicaments réglable (A101), est configurée pour :
- commander au dispositif de distribution (21) de distribuer les médicaments discrets (M1) identiques à l'aide du récipient de distribution de médicaments réglable (A101).

8. Pharmacie (1) selon la revendication 7, où le récipient de distribution de médicaments réglable (A101) comprend un mécanisme de distribution (109) avec un ou plusieurs corps de guidage (191, 193, 198) qui définissent des dimensions (X, X' ; Y, Y' ; Z, Z') d'au moins une partie du trajet de distribution (190) des médicaments discrets (M1) identiques à travers ledit mécanisme de distribution (109), où l'un ou plusieurs corps de guidage (191, 193, 198) sont réglables mécaniquement pour faire varier les dimensions (X, X' ; Y, Y' ; Z, Z') du trajet de distribution (190) défini par lesdits un ou plusieurs corps de guidage (191, 193, 198) entre le premier état de configuration et le deuxième état de configuration.

9. Procédé de distribution de médicaments discrets (M1, M2), où le procédé comprend les étapes suivantes :
- obtenir, par une unité de commande (6) pouvant être connectée à au moins un capteur (71-75), une valeur (V) d'un paramètre (P) indicatif d'une condition climatique ou de variations de taille et/ou de volume des médicaments discrets (M1, M2) résultant de ladite condition climatique, où la condition climatique concerne l'humidité et/ou la température dans un environnement dans lequel les médicaments discrets (M1, M2) sont stockés, remplis ou distribués (2, 3, 4), où le paramètre est l'humidité absolue (H1) ou l'humidité relative (H2) ; et où la valeur (V) du paramètre (P) est obtenue par l'une des méthodes suivantes : mesurer, par un capteur (71-75), l'humidité absolue (H1) directement, mesurer, par un capteur (71-75), l'humidité relative (H2) directement et calculer l'humidité absolue (H1) sur la base de la valeur mesurée (V) de l'humidité relative (H2) et d'une température (T) associée à ladite humidité relative (H2), ou se baser sur ou dériver de données météorologiques (W) d'une base de données météorologique (76) pouvant être connectée à l'unité de commande (6) ;
- déterminer, par l'unité de commande (6), si la valeur (V) du paramètre (P) est comprise dans une première plage (R1) ou une deuxième plage (R2) différente de la première plage (R1) ; et
- choisir, par l'unité de commande (6), pour des médicaments discrets (M1) identiques qui sont sujets à une expansion hygroscopique, entre l'utilisation d'un premier récipient de distribution de médicaments (A1) lorsque la valeur (V) du paramètre (P) est comprise dans la première plage (R1), et l'utilisation d'un deuxième récipient de distribution de médicaments (A2) lorsque la valeur (V) du paramètre (P) est comprise dans la deuxième plage (R2) , afin de distribuer les médicaments discrets (M1) identiques, où le deuxième récipient de distribution de médicaments (A2) a des dimensions différentes de celles du premier récipient de distribution de médicaments (A1);
- remplir un récipient de distribution de médicaments (A2) choisi parmi le premier récipient de distribution de médicaments (A1) et le deuxième récipient de distribution de médicaments (A2) avec les médicaments discrets (M1) identiques ; et
- distribuer, par un dispositif de distribution (21), les médicaments discrets (M1) identiques à l'aide du récipient de distribution de médicaments (A2) choisi.

10. Procédé selon la revendication 9, où la valeur (V) du paramètre (P) est mesurée, par l'au moins un capteur (72) dans un environnement de distribution (2) dans lequel les médicaments discrets (M1) identiques sont distribués ; et/ou où la valeur (V) du paramètre (P) est mesurée, par l'au moins un capteur (73, 74), dans un environnement de stockage (3) et/ou un environnement de remplissage (4) dans lequel les médicaments discrets (M1) identiques sont stockés et/ou remplis, respectivement.

11. Procédé selon la revendication 9, où la valeur (V) du paramètre (P) est un temps calendaire (D), en particulier une date calendaire, un mois civil, un trimestre civil ou une autre période de temps liée au calendrier ; et/ou où la valeur (V) du paramètre (P) est du temps passé par les médicaments discrets (M1, M2) identiques dans l'environnement dans lequel les médicaments discrets (M1, M2) sont stockés, remplis ou distribués (2, 3, 4).

12. Procédé selon l'une quelconque des revendications 9 à 11, où le procédé comprend l'étape d'assignation, par l'unité de commande (6), du premier récipient de distribution de médicaments (A1) et du deuxième récipient de distribution de médicaments (A2) aux médicaments discrets (M1) identiques, avant l'étape de choix entre le premier récipient de distribution de médicaments (A1) et le deuxième récipient de distribution de médicaments (A2).

13. Procédé selon l'une quelconque des revendications 9 à 12, où le procédé comprend en outre l'étape d'affichage, par une interface homme-machine (81, 82), du choix (C) effectué lors de l'étape de choix entre le premier récipient de distribution de médicaments (A1) et le deuxième récipient de distribution de médicaments (A2), de préférence, où le choix affiché (C) est une instruction à un opérateur de remplir le récipient de distribution de médicaments (A2) choisi avec les médicaments discrets (M1) identiques.

14. Procédé selon l'une quelconque des revendications 9 à 13, où le premier récipient de distribution de médicaments (A1) et le deuxième récipient de distribution de médicaments (A2) comprennent chacun un mécanisme de distribution (9, 9') avec un ou plusieurs corps de guidage (91, 93, 95 ; 91', 93', 95') qui définissent des dimensions (X, X' ; Y, Y' ; Z, Z') d'au moins une partie d'un trajet de distribution (90, 90') des médicaments discrets (M1) identiques à travers ledit mécanisme de distribution (9, 9'), où les dimensions (X', Y', Z') du trajet de distribution (90') défini par lesdits un plusieurs corps de guidage (91', 93', 95') du deuxième récipient de distribution de médicaments (A2) sont différentes des dimensions (X, Y, Z) du trajet de distribution (90) défini par lesdits un ou plusieurs corps de guidage (91, 93, 95) du premier récipient de distribution de médicaments (A1), de préférence, où les dimensions du trajet de distribution (90, 90') défini par lesdits un plusieurs corps de guidage (91, 93, 95 ; 91', 93', 95') sont fixées pour le premier récipient de distribution de médicaments (A1) et le deuxième récipient de distribution de médicaments (A2).

15. Procédé de distribution de médicaments discrets (M1, M2), où le procédé comprend les étapes suivantes :
- obtenir, par une unité de commande (6), une valeur (V) d'un paramètre (P) indicatif d'une condition climatique ou de variations de taille et/ou de volume des médicaments discrets (M1, M2) résultant de ladite condition climatique, où la condition climatique concerne l'humidité et/ou la température dans un environnement dans lequel les médicaments discrets (M1, M2) sont stockés, remplis ou distribués (2, 3, 4), où le paramètre est l'humidité absolue (H1) ou l'humidité relative (H2) ; et où la valeur (V) du paramètre (P) est obtenue par l'une des méthodes suivantes : mesurer, par un capteur (71-75), l'humidité absolue (H1) directement, mesurer, par un capteur (71-75), l'humidité relative (H2) directement et calculer l'humidité absolue (H1) sur la base de la valeur mesurée (V) de l'humidité relative (H2) et d'une température (T) associée à ladite humidité relative (H2), ou se baser sur ou dériver de données météorologiques (W) d'une base de données météorologique (76) pouvant être connectée à l'unité de commande (6) ;
- déterminer, par l'unité de commande (6), si la valeur (V) du paramètre (P) est comprise dans une première plage (R1) ou une deuxième plage (R2) différente de la première plage (R1) ;
- générer, par l'unité de commande (6), une instruction de réglage des dimensions (X, X' ; Y, Y' ; Z, Z') d'un récipient de distribution de médicaments réglable (A101), pour des médicaments discrets (M1) identiques qui sont soumis à une expansion hygroscopique, où le récipient de distribution de médicaments réglable (A101) est réglé sur un premier état de configuration avec des premières dimensions (X, Y, Z) lorsque la valeur (V) du paramètre (P) est comprise dans la première plage (R1) et réglé sur un deuxième état de configuration avec des deuxièmes dimensions (X', Y', Z'), différentes des premières dimensions (X, Y, Z), lorsque la valeur (V) du paramètre (P) est comprise dans la deuxième plage (R2) ;
- distribuer, par un dispositif de distribution (21), les médicaments discrets (M1) identiques à l'aide du récipient de distribution de médicaments réglable (A101).

16. Procédé selon la revendication 15, où le récipient de distribution de médicaments réglable (A101) comprend un mécanisme de distribution (109) avec un ou plusieurs corps de guidage (191, 193, 198) qui définissent des dimensions d'au moins une partie d'un trajet de distribution (190) des médicaments discrets (M1) identiques à travers ledit mécanisme de distribution (109), où l'un ou plusieurs corps de guidage (191, 193, 198) sont mécaniquement réglables pour faire varier les dimensions (X, X' ; Y, Y' ; Z, Z') du trajet de distribution (190) défini par lesdits un ou plusieurs corps de guidage (191, 193, 198) entre le premier état de configuration et le deuxième état de configuration ; et/ou où le récipient de distribution de médicaments réglable (A101) comprend un élément actionneur (199) afin de régler mécaniquement l'un ou plusieurs corps de guidage (191, 193, 198), où l'élément actionneur (199) comprend un matériau hygroscopiquement expansible.

17. Récipient de distribution de médicaments (A101) pour l'utilisation dans une pharmacie (1), où le récipient de distribution de médicaments (A101) comprend un mécanisme de distribution (109) avec un ou plusieurs corps de guidage (191, 193, 198) qui sont réglables mécaniquement entre un premier état de configuration avec des premières dimensions (X, Y, Z) et un deuxième état de configuration avec des deuxièmes dimensions (X', Y', Z'), différentes des premières dimensions (X, Y, Z), où le récipient de distribution de médicaments (A101) comprend un élément actionneur (199) afin de régler mécaniquement l'un ou plusieurs corps de guidage (191, 193, 198), où l'élément actionneur (199) comprend un matériau hygroscopiquement expansible.

18. Produit de programme informatique comprenant un support lisible par ordinateur non transitoire (62) contenant des instructions qui, lorsqu'elles sont exécutées par un processeur (61), amènent une pharmacie (1, 100) selon l'une quelconque des revendications 1 à 8 à exécuter les étapes du procédé selon l'une quelconque des revendications 9 à 17.
